# EUROPEAN PATENT APPLICATION

(11) **EP 4 686 726 A1**
(43) Date of publication of application: **04.02.2026**
(21) Application number: 24306282.5
(22) Date of filing: 30.07.2024
(51) Int. Cl.: C07K 14/47, C12N 15/70, C12N 15/74, D01F 9/00

(54) **MICROBIAL PRODUCTION OF RECOMBINANT MINK HAIR KERATINS FOR TEXTILE APPLICATIONS**

(71) Applicant: Fendi s.r.l., 00144 Roma (RM) (IT)
(72) Inventor: Püllmann, Pascal, LONDON, SW7 2AZ (GB); Van de Steen, Alexander, LONDON, SW7 2AZ (GB); Ellis, Thomas Marc, LONDON, SW7 2AZ (GB); COLLET, Carole, LONDON, N1C 4AA (GB)
(74) Representative: Regimbeau

(57) **Abstract**

The present invention concerns an isolated recombinant nucleic acid molecule comprising at least one nucleic acid sequence encoding at least one mink hair keratin protein and further comprising a heterologous promoter controlling the expression of said at least one nucleic acid sequence, as well as an expression vector comprising said nucleic acid molecule, a host cell comprising said nucleic acid molecule or said expression vector and a method of production of mink hair keratin protein using said nucleic acid molecule.

## Description

### FIELD OF THE INVENTION

The present invention concerns the production of recombinant mink hair keratin proteins for textile applications.

### INTRODUCTION

Natural fur is derived from animal pelts that constitute underlying skin tissue and overlaying keratinous cuticular hairs. It is naturally a high-performance insulative material that is beautiful in appearance. Animals such as mink, rabbit, cattle and sheep are the main sources of natural fur raw materials. However, in recent years, artificial fur is becoming more and more popular due to animal welfare concerns and cost of production.

Unlike natural furs that are limited in supply, artificial furs, of which the majority are of petrochemical origins, can be mass-produced. In recent years, artificial furs have attained wide use, for example, in clothing, accessories (such as bags), carpets, and stuffed animals. Most artificial furs available so far are manufactured from synthetic fibres, which are derived from petroleum and may use large amounts of energy during manufacture and are inherently non-biodegradable, constituting to the global plastic waste challenge.

Therefore, current artificial synthetic furs are not satisfactory for environmental reasons. Moreover, the physical and textural properties of artificial synthetic furs do not perform in a comparable way to natural furs, limiting their applications as textiles.

There is thus still a need for new alternatives to natural furs and artificial synthetic furs which avoid the ethical and/or environmental problems linked to the use of these materials.

Alpha-keratins are a type of protein intermediate filament produced by epithelial cells in vertebrates. They constitute to cuticular tissues including hair, fur, the outer layer of skin, horns, nails, wool, feathers, claws and hooves.

In order to mimic natural mink fur as closely as possible, the present inventors proposed to microbially-produce mink fur keratin, which would be used as the initial component for the production of a recombinant mink fur fibre. Such a fur replacement material would have benefits over natural fur for ethical and environmental reasons, whilst having more closely related physical and haptic properties to natural fur.

However, no mink fur keratin proteins have been recombinantly produced so far and keratins are not endogenous in microbial phyla such as yeast and bacteria.

The human genome contains about 54 different keratin genes, many of which are expressed exclusively in various compartments of hair follicles. There are 28 types of keratins in human epithelial cells, of which 17 types of keratin proteins that are specifically located in hair.

The present invention results from the demonstration, by the present inventors, of the recombinant production of Mink (*Neogale vison)* keratin, in particular modified Mink keratin, in microbial phyla, in particular in *Escherichia coli* and *Komagataella phaffii* hosts.

Specifically, to achieve the present invention, the present inventors succeeded in (i) identifying critical homologous mink cuticular keratin genes from expression characteristics from human hair, (ii) refactoring and modifying the genes for microbial expression and purification, and (iii) demonstrating viable expression of each keratin candidate in both *Escherichia coli* and *Komagataella phaffii* hosts.

### SUMMARY OF THE INVENTION

The present invention thus arises from the identification of key keratin genes within *Neogale vison* genome, followed by the modification and optimisation of these identified genes DNA sequences for the detection, production and purification of the resulting proteins in bacterial and yeast hosts.
The modifications of the identified genes DNA sequences include for example: genetic construction of the keratin gene open reading frame under appropriate host control elements; refactorization of the DNA coding sequence for specific host expression; genetic fusion of a single or repeated hexahistine residue sequence as an amino or carboxyl-terminal addition; amino-terminal addition of a set of peptide sequences, in yeast constructs, for the secretion of recombinant keratin for industrial-scale production; and site-specific mutations to alter post-translational modification within the host secretory pathway

The present invention thus concerns an isolated recombinant nucleic acid molecule comprising at least one nucleic acid sequence encoding at least one mink hair keratin protein and further comprising a heterologous promoter controlling the expression of said at least one nucleic acid sequence.

Another object of the invention concerns an expression vector comprising the nucleic acid molecule of the invention.

The present invention further concerns a host cell comprising the nucleic acid of the invention or the expression vector of the invention.

The present invention also relates to a kit for producing recombinant mink hair keratin protein comprising:
a) a host cell, and
b) a nucleic acid of the invention or an expression vector of the invention.

Still another object of the invention concerns a method for producing recombinant mink hair keratin protein, comprising:
a) providing a host cell comprising the nucleic acid of the invention or the expression vector of the invention, and
b) culturing said host cell under conditions and for a period of time enabling the production of said mink hair keratin protein by said host cell.

The present invention also concerns a recombinant mink hair keratin protein obtainable by the method of production of the invention.

Another object of the invention concerns a recombinant protein comprising a recombinant mink hair keratin protein sequence, and further comprising:
- a secretion signal at the N-terminal end of said mink hair keratin protein sequence and/or
- a tag at the N-terminal and/or C-terminal end of said mink hair keratin protein sequence.

### DETAILED DESCRIPTION OF THE INVENTION

### Definitions

The singular forms "a," "an," and "the," are used in the sense that they include plural reference of the referenced components or steps, such as "at least one", "at least a first", "one or more" or "a plurality", unless the context clearly dictates otherwise. Thus, for example, a reference to "a cell" or "a microorganism" includes a plurality of cells or microorganisms, including mixtures thereof.

The terms "comprise", "contain", "include" and variations thereof such as "comprising" are used herein in an inclusive sense, i.e., to specify the presence of the stated features but meaning that any further such features can be present in various embodiments of the invention.

The terms "consist essentially of", "consist essentially in" and variations thereof such as "consisting essentially of" or "consisting essentially in" are used to specify the presence of the stated features but meaning that specific further such features that not materially affect the essential characteristics of the invention can be present in various embodiments of the invention.

The terms "consist", and variations thereof such as "consisting of" or "consisting in" are used in an exclusive sense, i.e., to specify the presence of the stated features meaning that no further can be present in various embodiments of the invention.

A "nucleic acid" as used herein refers to a single- or double-stranded linear polynucleotide containing deoxyribonucleotides and/or ribonucleotides that are linked by 3'-5'-phosphodiester bonds. A "nucleic acid" refers to either DNA or RNA, single-stranded or double-stranded, and any chemical modifications thereof. By "nucleic/acid", such hybrid molecules as DNA/RNA duplexes, i.e., a molecule comprising a DNA strand and a complementary RNA strand, are also encompassed. The terms "deoxyribonucleic acid" and "DNA" as used herein mean a polymer composed of deoxyribonucleotides. The terms "ribonucleic acid" and "RNA" as used herein mean a polymer composed of ribonucleotides. Modifications include, but are not limited to, those which provide other chemical groups that incorporate additional charge, polarisability, hydrogen bonding, electrostatic interaction, and functionality to the nucleic acid. Such modifications include, but are not limited to, 2'-position sugar modifications, 5-position pyrimidine modifications, 8-position purine modifications, modifications at exocyclic amines, substitution of 4-thiouridine, substitution of 5-bromo or 5-iodo-uracil; backbone modifications, methylations, unusual base-pairing combinations such as the isobases isocytidine and isoguanidine and the like. Examples of modified nucleotides include, but are not limited to 2,6-Diaminopurine (2-Amino-dA), 5-Methyl dC, locked nucleic acid (LNA), which are nucleotides in which the ribose moiety is modified with an extra bridge connecting the 2' oxygen and 4' carbon, unlocked nucleic acids (UNAs), which are acyclic RNA analogues without a C2'-C3' bond in the ribose ring, and peptide nucleic acid (PNA), wherein the backbone is composed of repeating N-(2-aminoethyl)-glycine units linked by peptide bonds. Modifications can also include 3' and 5' modifications such as capping.

For the purposes of this invention, by "isolated" is meant material that has been removed from its natural state or otherwise been subjected to human manipulation. Isolated material may be substantially or essentially free from components that normally accompany it in its natural state, or may be manipulated so as to be in an artificial state together with components that normally accompany it in its natural state. Isolated material may be in native, chemical, synthetic or recombinant form.

"Isolated" or "purified" notably refers in relation to a nucleic acid to a nucleotide polymer in the form of a separate fragment or as a component of a larger nucleic acid construct, which has been derived from nucleic acid isolated from its natural environment at least once. An "isolated nucleic acid" refers to a nucleic acid segment or fragment, which has been separated from sequences, which flank it in a naturally occurring state, e.g., a DNA fragment that has been removed from the sequences, which are normally adjacent to the fragment, e.g., the sequences adjacent to the fragment in a genome in which it naturally occurs.

As used herein, the term "recombinant" refers to nucleic acid synthesized or otherwise manipulated *in vitro* ("recombinant nucleic acid") and to methods of using recombinant nucleic acids to produce gene products encoded by those nucleic acids in cells or other biological systems. For example, a cloned nucleic acid may be inserted into a suitable expression vector, such as a bacterial plasmid, and the plasmid can be used to transform a suitable host cell. A host cell that comprises the recombinant nucleic acid is referred to as a "recombinant host cell". The gene is then expressed in the recombinant host cell to produce, e.g., a "recombinant protein".

As used herein, the terms "protein", "proteins", "polypeptide", and "polypeptides", are synonyms and refer to polymers of amino acids covalently linked through peptide bonds into a chain. Peptide bonds are formed between the carboxyl group of one amino acid and the amino group of the next amino acid. The terms also apply to amino acid polymers in which one or more amino acids are chemical analogues or modified derivatives of corresponding naturally-occurring amino acids. The terms "amino acids" and "amino acid" refer to all naturally occurring alpha amino acids in both their D and L stereoisomeric forms, and their analogues and derivatives. An analogue is defined as a substitution of an atom in the amino acid with a different atom that usually has similar properties. A derivative is defined as an amino acid that has another molecule or atom attached to it. Derivatives would include, for example, acetylation of an amino group, amination of a carboxyl group, or oxidation of the sulfur residues of two cysteine molecules to form cystine.

The percent identities referred to in the context of the disclosure of the present invention are determined after optimal alignment of the sequences to be compared, which may therefore comprise one or more insertions, deletions, truncations and/or substitutions.

This percent identity may be calculated by any sequence analysis method well-known to the person skilled in the art.

The percent identity may be determined after global alignment of the sequences to be compared over their entire length. In addition to manual comparison, it is possible to determine global alignment using the algorithm of Needleman and Wunsch (1970). For nucleotide sequences, the sequence comparison may be performed using any software well-known to a person skilled in the art, such as the Needle software. The parameters used may notably be the following: "Gap open" equal to 10.0, "Gap extend" equal to 0.5, and the EDNAFULL matrix (NCBI EMBOSS Version NUC4.4).

For amino acid sequences, the sequence comparison may be performed using any software well-known to a person skilled in the art, such as the Needle software. The parameters used may notably be the following: "Gap open" equal to 10.0, "Gap extend" equal to 0.5, and the BLOSUM62 matrix.

Preferably, the percent identify as defined in the context of the present invention is determined via the global alignment of sequences compared over their entire length.

By "heterologous" is meant, in the context of the invention, that the nucleic acid originates from a foreign source or, if from the same source, is modified from its original form. Thus, a "heterologous promoter" is a promoter not normally associated with the nucleic acid sequence encoding a mink hair keratin protein of the present invention.

By "homologous" is meant, in the context of the invention, a direct relationship among a "family" of genes in which certain sequences or domains are strongly conserved among the members of the family.

As used herein, the term "expression" refers to the transcription and stable accumulation of sense (mRNA) or anti-sense RNA derived from a nucleic acid, and/or to translation of an mRNA into a polypeptide

### Recombinant nucleic acid molecule encoding a mink hair keratin protein

The present invention concerns an isolated recombinant nucleic acid molecule comprising at least one nucleic acid sequence encoding at least one mink hair keratin protein and further comprising a heterologous promoter controlling the expression of said at least one nucleic acid sequence.

The present inventors indeed identified genes encoding hair keratin protein in mink and succeeded in cloning these genes in bacterial and yeast vectors and expressing them in bacteria and yeast to efficiently produce mink hair keratin proteins in these microorganisms.

By "hair keratin protein" is meant herein a structural fibrous protein typically making up scales, hair, nails, feathers, horns, claws, hooves, and the outer layer of skin among vertebrates.

By "mink hair keratin protein" is meant herein a hair keratin protein originally produced by mink cells.

By "mink" is meant herein a dark-colored, semiaquatic, carnivorous mammal of the genera *Neogale* and *Mustela* and part of the family *Mustelidae.* Minks include in particular animals from the species *Neogale vison* and *Mustela lutreola.* In a particular embodiment, said mink hair keratin protein is a *Neogale vison* hair keratin protein.

In a particular embodiment, said mink hair keratin protein is a mink pre-cortex keratin protein.

As used herein, the terms "pre-cortex keratin protein", "cortex keratin protein", "pre-cortical keratin protein" and "cortical keratin protein" are used interchangeably, and refer to keratin proteins produced by cells of hair cortex or hair pre-cortex, such as cortical cells or pre-cortical cells of hair, or to keratin proteins homologous to keratin proteins known to be produced by cells of hair cortex or hair pre-cortex, in particular cells of human hair cortex or pre-cortex, such as cortical cells or pre-cortical cells of hair, in particular of human hair.

In a particular embodiment, said mink hair keratin protein, which is typically a mink pre-cortex keratin protein, comprises, or consists of, an amino acid sequence at least 80% identical to a sequence selected from the group consisting of SEQ ID NO: 1 [KRT31], SEQ ID NO: 2 [KRT35], SEQ ID NO: 3 [KRT81] and SEQ ID NO: 4 [KRT85]. In a more particular embodiment, said mink hair keratin protein, which is typically a mink pre-cortex keratin protein, comprises, or consists of, an amino acid sequence at least 85%, at least 90%, at least 95%, at least 96%, at least 97%, at least 98%, or at least 99% identical to a sequence selected from the group consisting of SEQ ID NO: 1 [KRT31], SEQ ID NO: 2 [KRT35], SEQ ID NO: 3 [KRT81] and SEQ ID NO: 4 [KRT85]. In still a particular embodiment, said mink hair keratin protein, which is typically a mink pre-cortex keratin protein, comprises, or consists of, an amino acid sequence selected from the group consisting of SEQ ID NO: 1 [KRT31], SEQ ID NO: 2 [KRT35], SEQ ID NO: 3 [KRT81] and SEQ ID NO: 4 [KRT85].

In a particular embodiment, said mink hair keratin protein, which is typically a mink pre-cortex keratin protein, comprises, or consists of, an amino acid sequence at least 80% identical to the sequence SEQ ID NO: 3 [KRT81]. In a more particular embodiment, said mink hair keratin protein, which is typically a mink pre-cortex keratin protein, comprises, or consists of, an amino acid sequence at least 85%, at least 90%, at least 95%, at least 96%, at least 97%, at least 98%, or at least 99% identical to the sequence SEQ ID NO: 3 [KRT81]. In still a particular embodiment, said mink hair keratin protein, which is typically a mink pre-cortex keratin protein, comprises, or consists of, the sequence SEQ ID NO: 3 [KRT81].

In another particular embodiment, said mink hair keratin protein is a mink cuticular keratin protein.

As used herein, the terms "cuticular keratin protein" and "cuticle keratin protein" are used interchangeably, and refer to keratin proteins produced by cells of hair cuticle, such as cuticular cells of hair, or to keratin proteins homologous to keratin proteins known to be produced by cells of hair cuticle, in particular cells of human hair cuticle, such as cuticular cells of hair, in particular of human hair.

In a particular embodiment, said mink hair keratin protein, which is typically a mink cuticular keratin protein, comprises, or consists of, an amino acid sequence at least 80% identical to a sequence selected from the group consisting of SEQ ID NO: 5 [KRT32] and SEQ ID NO: 6 [KRT82]. In a more particular embodiment, said mink hair keratin protein, which is typically a mink cuticular keratin protein, comprises, or consists of, an amino acid sequence at least 85%, at least 90%, at least 95%, at least 96%, at least 97%, at least 98%, or at least 99% identical to a sequence selected from the group consisting of SEQ ID NO: 5 [KRT32] and SEQ ID NO: 6 [KRT82]. In still a particular embodiment, said mink hair keratin protein, which is typically a mink cuticular keratin protein, comprises, or consists of, an amino acid sequence selected from the group consisting of SEQ ID NO: 5 [KRT32] and SEQ ID NO: 6 [KRT82].

The nucleic acid molecule of the invention further comprises a heterologous promoter controlling the expression of said at least one nucleic acid sequence.

A "promoter" as used herein, refers to a nucleotide sequence that is capable of controlling the expression of a coding sequence or gene. Promoters are generally located 5' of the sequence that they regulate. Promoters may be derived in their entirety from a native gene, or be composed of different elements derived from promoters found in nature, and/or comprise synthetic nucleotide segments. Those skilled in the art will readily ascertain that different promoters may regulate expression of a coding sequence or gene in response to a particular stimulus, e.g., in a cell-specific or tissue-specific manner, in response to different environmental or physiological conditions, or in response to specific compounds.

As will be understood by the skilled person, the choice of the heterologous promoter will depend on the host cell in which the nucleic acid sequence encoding the at least one mink hair keratin protein will be expressed. For example, when the nucleic acid sequence encoding the mink hair keratin protein is to be expressed in a bacterial cell, said heterologous promoter will be selected from bacteria-specific promoters. Alternatively, when the nucleic acid sequence encoding the mink hair keratin protein is to be expressed in a yeast cell, said heterologous promoter will be selected from yeast-specific promoters.

Examples of bacteria-specific promoters are well-known from the skilled person and include the *Escherichia coli* lac or trp promoters, the lacl promoter, the lacZ promoter, the T3 promoter, the T7 promoter, the gpt promoter, the lambda PR promoter, the lambda PL promoter, promoters from operons encoding glycolytic enzymes such as 3-phosphoglycerate kinase (PGK), and the acid phosphatase promoter. In a particular embodiment, when said nucleic acid molecule is for expression in bacteria, said promoter is preferably the T7 promoter.

Examples of yeast-specific promoters are well-known from the skilled person and include pCYC1, pADH1, pSTE5, pADH1, pCYC100 minimal, pCYC70 minimal, pCYC43 minimal, pCYC28 minimal, pCYC16, pPGK1, pCYC, pGPD, pTDH3, pGAL1, pMFA1, pMFA2, pSTE3, pURA3, pFIG1, pENO2, pDLD, pJEN1, pmCYC, pFMD, pGAP and pSTE2. In a particular embodiment, when said nucleic acid molecule is for expression in yeasts, said promoter is preferably the FMD promoter.

Promoters are typically classified into two classes: inducible and constitutive.

"Constitutive promoter" refers to a promoter that is capable of facilitating continuous transcription of a coding sequence or gene under its control and/or to which it is operably linked. Constitutive promoters and variants are well known in the art.

An "inducible promoter" refers to a promoter that initiates increased levels of transcription of the coding sequence or gene under its control in response to a stimulus or an exogenous environmental condition. A "directly inducible promoter" refers to a regulatory region, wherein the regulatory region is operably linked to a gene encoding a protein or polypeptide, where, in the presence of an inducer of said regulatory region, the protein or polypeptide is expressed. An "indirectly inducible promoter" refers to a regulatory system comprising two or more regulatory regions, for example, a first regulatory region that is operably linked to a first gene encoding a first protein, polypeptide, or factor, e.g., a transcriptional regulator, which is capable of regulating a second regulatory region that is operably linked to a second gene, the second regulatory region may be activated or repressed, thereby activating or repressing expression of the second gene. Both a directly inducible promoter and an indirectly inducible promoter are encompassed by "inducible promoter." For example, and without limitation, chemical agents, temperature, and light may be used for induction of the promoters contemplated herein.

In a preferred embodiment, said heterologous promoter is an inducible promoter.

Examples of inducible promoters are well-known from the skilled person. Examples of bacteria-specific inducible promoters include FNR promoter, ParaC promoter, ParaBAD promoter, propionate promoter, and PTetR promoter. Examples of yeast-specific inducible promoters include pGAL1, pMFA1, pMFA2, pSTE3, pURA3, pFIG1, pENO2, pDLD, pJEN1, pmCYC, pFMD and pSTE2.

In the context of the present invention, said heterologous promoter is operably linked to said nucleic acid sequence encoding at least one mink hair keratin protein.

As used herein, "operably linked" means that the elements being linked are arranged so that they function in concert for their intended purposes. For example, a promoter is operably linked to a nucleic acid molecule if the promoter effects transcription from the transcription initiation to the terminator of said nucleic acid molecule in a permissive host cell.

In a particular embodiment, the nucleic acid molecule further comprises a terminator.

By "terminator" is meant herein a DNA sequence at the end of a transcriptional unit which signals termination of transcription. Examples of terminators are well-known from the skilled person and include trp, tac, lac, trc, APL, T7, tpA, lpp, and T4 terminators. In a particular embodiment, said terminator is a T7 terminator.

In a particular embodiment, said nucleic acid sequence is codon-optimized for prokaryotic expression and/or yeast expression.

By "codon-optimized" is meant herein to modify a coding nucleic sequence to include codons that are optimized for expression in a designated host cell. Redundancy in the genetic code allows some amino acids to be encoded by more than one codon, but certain codons are less "optimal" than others because of the relative availability of matching tRNAs as well as other factors. Codon optimized sequences can be synthetic sequences, and encode the identical polypeptide encoded by the non-codon optimized parent polynucleotide.

Preferably the nucleic acid sequence described herein is codon optimized for use in prokaryotes, such as bacteria and/or in yeasts.

In a particular embodiment, said nucleic acid sequence is codon-optimized for prokaryotic expression, in particular for expression in bacterial cells.

As used herein, the term "bacteria" or "bacterial cell" indicates a large domain of prokaryotic microorganisms. Typically, a few micrometers in length, bacteria have a number of shapes, ranging from spheres to rods and spirals, and are present in several habitats. Bacteria in the sense of the disclosure refers to several phyla notably including: Actinomycetota, Bacillota (formerly Firmicutes), Bacteroidota, Campylobacterota, Chlamydiota, Fusobacteriota, Mycoplasmatota, and Pseudomonadota (formerly Proteobacteria).

In a particular embodiment, said nucleic acid sequence is codon-optimized for expression in Proteobacteria bacteria. In a more particular embodiment, said nucleic acid sequence is codon-optimized for expression in Gammaproteobacteria bacteria, in particular for expression in Enterobacterales bacteria, more particularly for expression in Enterobacteriaceae bacteria.

In a particular embodiment, said nucleic acid sequence is codon-optimized for expression in bacteria of the genus *Escherichia.* In a more particular embodiment, said nucleic acid sequence is codon-optimized for *Escherichia coli* expression.

However, although *E. coli* was utilized in exemplary embodiments, the present disclosure is not limited to this genus and species of bacteria.

In another particular embodiment, said nucleic acid sequence is codon-optimized for yeast expression.

As used herein, the term "yeast", "yeast cell" or "fungal cell" refer to eukaryotic, single-celled microorganisms classified as members of the fungus kingdom. Examples of yeasts include Examples of such yeast host cell include yeasts belonging to the *Saccharomyces* genus, yeasts belonging to the *Pichia* genus, yeasts belonging to the *Komagataella* genus, yeasts belonging to the *Kluyveromyces* genus, yeasts belonging to the *Schizosaccharomyces* genus, yeasts belonging to the *Zygosacharomyces* genus, yeasts belonging to the *Yarrowia* genus, yeasts belonging to the *Candida* genus and yeasts belonging to the *Hansenula* genus.

In a particular embodiment, said nucleic acid sequence is codon-optimized for expression in yeasts of the *Saccharomyces* genus, yeasts of the *Pichia* genus or yeasts of the *Komagataella* genus. In a more particular embodiment, said nucleic acid sequence is codon-optimized for expression in yeasts of the *Komagataella* genus. In a more particular embodiment, said nucleic acid sequence is codon-optimized for *Komagataella phaffii* (formerly *Pichia pastoris*) expression.

However, although *Komagataella phaffii* was utilized in exemplary embodiments, the present disclosure is not limited to this genus and species of yeasts.

In a particular embodiment, said nucleic acid sequence is optimized or further optimized to remove glycosylation sites.

As used herein, the term "glycosylation site" refers to an amino acid residue which is recognized by a cell, in particular an eukaryotic cell, as location for the attachment of sugar residues. The amino acids where sugars are attached are typically asparagine (abbreviated Asn or N) (for N-linked sugars), threonine (abbreviated Thr or T) or serine (abbreviated Ser or S) (for O-linked sugars) residues. The specific site of attachment is typically signaled by a sequence of amino acids, e.g., Asn-X-(Thr or Ser) for most N-linked attachment and (Thr or Ser)-X-X-Pro for most O-linked attachment, where X is any amino acid. The terms N-linked and O-linked refer to the chemical group that serves as the attachment site between the sugar moiety and the amido acid residue. N-linked sugars are attached through an amino group; O-linked sugars are attached through an hydroxyl group.

Techniques to remove glycosylation sites are well-known from the skilled person and include substitution, for example by alanine residue replacement, of the asparagine, threonine or serine residue on which said sugar attach, or of one of the amino acids signaling the sugar attachment site, as defined above.

In a particular embodiment, the nucleic acid molecule of the invention further comprises a nucleic acid sequence encoding a tag at the N-terminal end and/or at the C-terminal end of the mink hair keratin protein.

By "tag" is meant herein an attached molecule or molecules useful for the identification or isolation of the attached component. Preferably, the tag is covalently bound to the attached component. In a particular embodiment, the tag is a protein tag. Protein tags include, but are not limited to, Avi tag, calmodulin tag, FLAG tag, HA tag, His tag, double His tag, Myc tag, c-myc tag, S tag, SBP tag, Softag 1, Softag 3, V5 tag, C9 tag, Xpress tag, Isopeptag, SpyTag, streptactin tag and the like. In a preferred embodiment, the tag is selected from the group consisting of FLAG tag, HA tag, His tag, double His tag and c-myc tag. In a more preferred embodiment, the tag is His tag.

In a particular embodiment, the tag, in particular the His tag, is at the N-terminal end of the mink hair keratin protein. In another particular embodiment, the tag, in particular the His tag or double His tag, is at the C-terminal end of the mink hair keratin protein.

In a particular embodiment, the nucleic acid molecule comprising at least one nucleic acid sequence encoding at least one mink hair keratin protein and a tag at the N-terminal end of the mink hair keratin protein encodes a protein which comprises, or consists of, an amino acid sequence at least 85%, at least 90%, at least 95%, at least 96%, at least 97%, at least 98%, or at least 99% identical to a sequence selected from the group consisting of SEQ ID NO: 7 ("His-KRT31 fusion protein"), SEQ ID NO: 8 ("His-KRT35 fusion protein"), SEQ ID NO: 9 ("His-KRT81 fusion protein"), SEQ ID NO: 10 ("His-KRT85 fusion protein"), SEQ ID NO: 11 ("His-KRT32 fusion protein") and SEQ ID NO: 12 ("His-KRT82 fusion protein"). In a more particular embodiment, the nucleic acid molecule comprising at least one nucleic acid sequence encoding at least one mink hair keratin protein and a tag at the N-terminal end of the mink hair keratin protein encodes a protein which comprises, or consists of, an amino acid sequence selected from the group consisting of SEQ ID NO: 7, SEQ ID NO: 8, SEQ ID NO: 9, SEQ ID NO: 10, SEQ ID NO: 11 and SEQ ID NO: 12.

In a particular embodiment, the nucleic acid molecule of the invention further comprises a nucleic acid sequence encoding a secretion signal at the N-terminal end of the mink hair keratin protein.

As used herein, the terms "signal peptide" and "secretion signal" are used interchangeably and refer to an amino acid sequence of a protein which when present results in the transportation and secretion of the protein to the exterior of the cell. Secretion signals are typically cleavable hydrophobic segments of a precursor protein at or near the N terminus of the precursor protein. In the secretion process, such secretion signals are enzymatically removed to result in the secretion of a mature form of the protein, i.e. a form of the protein lacking the secretion signal. Appropriate signal peptides are known in the art. Examples of secretion signals include malE, ompT, pelB, bacteriophage fd gene III protein signal, gill, CAT leader, SRP, OmpA signal peptide, α factor signal peptide, invertase 2 signal peptide, wild-type unspecific peroxygenase (UPO) signal peptide, killer protein signal peptide, secretion signal of the Kph-0030 protein and a genetic fusion of a pre-alpha sequence to Ost1 co-translocation signal.

In a particular embodiment, said secretion signal is selected from the group consisting of α factor signal peptide, invertase 2 signal peptide, wild-type UPO signal peptide, killer protein signal peptide, secretion signal of the Kph-0030 protein and a genetic fusion of a pre-alpha sequence to Ost1 co-translocation signal.

α factor signal peptide typically comprises or consists of the sequence SEQ ID NO: 13.

Invertase 2 signal peptide typically comprises or consists of the sequence SEQ ID NO: 14.

Wild-type UPO signal peptide typically comprises or consists of the sequence SEQ ID NO: 15.

Killer protein signal peptide typically comprises or consists of the sequence SEQ ID NO: 16.

The secretion signal of the Kph-0030 protein typically comprises or consists of the sequence SEQ ID NO: 17.

The genetic fusion of a pre-alpha sequence to Ost1 co-translocation signal typically comprises or consists of the sequence SEQ ID NO: 18.

In a particular embodiment, the nucleic acid molecule comprising at least one nucleic acid sequence encoding at least one mink hair keratin protein and further comprising a nucleic acid sequence encoding a secretion signal at the N-terminal end of the mink hair keratin protein encodes a protein which comprises, or consists of, an amino acid sequence at least 85%, at least 90%, at least 95%, at least 96%, at least 97%, at least 98%, or at least 99% identical to a sequence selected from the group consisting of SEQ ID NO: 19 ("preostalpha SP-KRT31"), SEQ ID NO: 20 ("See prepo alpha SP-KRT31"), SEQ ID NO: 21 ("Gma UPO SP-KRT32"), SEQ ID NO: 22 ("preostalpha SP-KRT82"), SEQ ID NO: 23 ("Killer protein SP-KRT85") , SEQ ID NO: 24 ("Killer protein SP-KRT35"), SEQ ID NO: 25 ("alpha factor SP-KRT81"), SEQ ID NO: 26 ("0030 SP-KRT31"), SEQ ID NO: 27 ("0030 SP-KRT32"), SEQ ID NO: 28 ("0030 SP-KRT82"), and SEQ ID NO: 29 ("Invertase 2 SP-KRT85"). In a more particular embodiment, the nucleic acid molecule comprising at least one nucleic acid sequence encoding at least one mink hair keratin protein and further comprising a nucleic acid sequence encoding a secretion signal at the N-terminal end of the mink hair keratin protein encodes a protein which comprises, or consists of, an amino acid sequence selected from the group consisting of SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, and SEQ ID NO: 29.

In a particular embodiment, the nucleic acid molecule comprises at least one nucleic acid sequence comprising at least one nucleic acid sequence encoding at least one mink hair keratin protein and a tag at the C-terminal end of the mink hair keratin protein and further comprises a nucleic acid sequence encoding a secretion signal at the N-terminal end of the mink hair keratin protein.

In a more particular embodiment, the nucleic acid molecule comprising at least one nucleic acid sequence encoding at least one mink hair keratin protein and a tag at the C-terminal end of the mink hair keratin protein and further comprising a nucleic acid sequence encoding a secretion signal at the N-terminal end of the mink hair keratin protein encodes a protein which comprises, or consists of, an amino acid sequence at least 85%, at least 90%, at least 95%, at least 96%, at least 97%, at least 98%, or at least 99% identical to a sequence selected from the group consisting of SEQ ID NO: 30 ("preostalpha SP-KRT31-His2"), SEQ ID NO: 31 ("See prepo alpha SP-KRT31-His2"), SEQ ID NO: 32 ("Gma UPO SP-KRT32-His2"), SEQ ID NO: 33 ("preostalpha SP-KRT82-His2"), SEQ ID NO: 34 ("Killer protein SP-KRT85-His2") , SEQ ID NO: 35 ("Killer protein SP-KRT35-His2"), SEQ ID NO: 36 ("alpha factor SP-KRT81-His2"), SEQ ID NO: 37 ("0030 SP-KRT31-His2"), SEQ ID NO: 38 ("0030 SP-KRT32-His2"), SEQ ID NO: 39 ("0030 SP-KRT82-His2"), and SEQ ID NO: 40 ("Invertase 2 SP-KRT85-His2"). In a more particular embodiment, the nucleic acid molecule comprising at least one nucleic acid sequence encoding at least one mink hair keratin protein and a tag at the C-terminal end of the mink hair keratin protein and further comprising a nucleic acid sequence encoding a secretion signal at the N-terminal end of the mink hair keratin protein encodes a protein which comprises, or consists of, an amino acid sequence selected from the group consisting of SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, and SEQ ID NO: 40.

### Expression vector

The present invention also concerns an expression vector comprising the nucleic acid as defined in the section *"Recombinant nucleic acid molecule encoding a mink hair keratin protein"* above.

The term "vector" as used herein refers to a vehicle, preferably a nucleic acid molecule or a viral particle that contains the elements necessary to allow delivery, propagation and/or expression of any of the nucleic acid molecule(s) described herein within a host cell. This term encompasses vectors for maintenance (cloning vectors) or vectors for expression in various host cells (expression vectors), extrachromosomal vectors (e.g. multicopy plasmids) or integration vectors (e.g. designed to integrate into the host cell genome and produce additional copies of the nucleic acid molecules when the host cell replicates) as well as shuttle vectors (e.g. functioning in both prokaryotic and/or eukaryotic hosts) and transfer vectors (e.g. for transferring nucleic acid molecule(s) in a viral genome). For the purpose of the invention, the vectors may be of naturally occurring genetic sources, synthetic or artificial, or some combination of natural and artificial genetic elements.

In the context of the invention, the term "vector" has to be understood broadly as including mRNA, plasmid and viral vectors. Vectors which are appropriate in the context of the present invention, include, without limitation, bacteriophage, plasmid or cosmid vectors for expression in prokaryotic host cells such as bacteria; vectors for expression in yeast; baculovirus vectors for expression in insect cell systems; as well as plasmid and viral vectors for expression in higher eukaryotic cells. Typically, such vectors are commercially available (e.g. in Invitrogen, Stratagene, Amersham Biosciences, Promega, etc.) or available from depositary institutions such as the American Type Culture Collection (ATCC, Rockville, Md.) or have been the subject of numerous publications describing their sequence, organization and methods of producing, allowing the artisan to apply them. The present invention also encompasses vectors (e.g. plasmid DNA and mRNA) complexed to lipids or polymers to form particulate structures such as liposomes, lipoplexes or nanoparticles.

In a particular embodiment, the vector of the invention is a plasmid.

A "plasmid vector" as used herein refers to a replicable DNA construct. Usually, plasmid vectors contain selectable marker genes that allow host cells carrying the plasmid vector to be selected for or against in the presence of a corresponding selective drug. A variety of positive and negative selectable marker genes are known in the art. By way of illustration, an antibiotic resistance gene can be used as a positive selectable marker gene that allows a host cell to be selected in the presence of the corresponding antibiotic.

In particular embodiments, the vector of the invention further comprises regulatory elements.

As used herein, the term "regulatory elements" or "regulatory sequence" refers to any element that allows, contributes or modulates the expression of nucleic acid molecule(s) in a given host cell or subject, including replication, duplication, transcription, splicing, translation, stability and/or transport of the nucleic acid(s) or its derivative (i.e. mRNA). It will be appreciated by those skilled in the art that the choice of the regulatory sequences can depend on such factors as the vector itself and the cells to be transformed, and will be easily selected by those skilled in the art based on common general knowledge and publications on this topic.

### Host cell

The present invention also concerns a host cell comprising the nucleic acid as defined in the section *"Recombinant nucleic acid molecule encoding a mink hair keratin protein"* above or the expression vector as defined in the section *"Expression vector"* above.

### Host cell

The term "host cell" refers to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

In the context of the invention, the host cell is not a mink cell.

In a particular embodiment, said host cell is a bacterial cell.

Any culturable bacterial cell is suitable to be used as host cell in the context of the invention. Examples of suitable host bacterial cells include bacteria from the Actinomycetota, Bacillota (formerly Firmicutes), Bacteroidota, Campylobacterota, Chlamydiota, Fusobacteriota, Mycoplasmatota, and Pseudomonadota (formerly Proteobacteria) phyla. In particular embodiments, said host cell is a Proteobacteria bacterial cell, more particularly a Gammaproteobacteria bacterial cell, still more particularly an Enterobacterales bacterial cell, most particularly an Enterobacteriaceae bacterial cell.

In a particular embodiment, said host bacterial cell is a bacterial cell of the genus *Escherichia,* more particularly an *Escherichia coli* bacterial cell.

The *Escherichia coli* bacterial cell may be for example C41(DE3), CLEARCOLI BL21(DE3), CMR300, BL21(DE3), BL21(DE3)pLysS, BL21-Gold(DE3), BL21-Gold(DE3)pLysS, ROSETTA^{™} (DE3), ROSETTA^{™} (DE3)pLysS, ROSETTA^{™} 2(DE3), ROSETTA^{™} 2(DE3)pLysS, ROSETTA^{™} 2(DE3)pLacI, ROSETTA^{™} (DE3)pLacI, C43(DE3), W3110, MG1655, MC1000, BW25113, or DH5α. For example, the *Escherichia coli* may be a BL21(DE3) strain.

In a particular embodiment, in particular when said host cell is a bacterial cell, said at least one nucleic acid molecule or said expression vector is episomal.

By "episomal" is meant herein that the vector is an autonomously replicating element within the host cell. Autonomously replicating elements include for examples plasmids or cosmids.

In another particular embodiment, said host cell is a yeast.

Any culturable yeast cell is suitable to be used as host cell in the context of the invention. Examples of suitable host yeast cells include yeasts belonging to the *Saccharomyces* genus, yeasts belonging to the *Pichia* genus, yeasts belonging to the *Komagataelia* genus, yeasts belonging to the *Kluyveromyces* genus, yeasts belonging to the *Schizosaccharomyces* genus, yeasts belonging to the *Zygosacharomyces* genus, yeasts belonging to the *Yarrowia* genus, yeasts belonging to the *Candida* genus and yeasts belonging to the *Hansenula* genus. In particular embodiments, said host cell is a yeast of the *Saccharomyces* genus, of the *Pichia* genus or of the *Komagataella* genus.

In a particular embodiment, said host cell is a yeast of the *Komagataella* genus, more particularly a *Komagataella phaffii* (formerly *Pichia pastoris)* cell.

The *Komagataella phaffii* cell may be for example Y-11430, NCYC 2543, X-33, GS115, KM71, KM71H, SMD1168, SMD1168H, SMD1165, or MC100-3. For example, the *Komagataella phaffii* may be a X-33 strain.

In a particular embodiment, in particular when said host cell is a yeast, said at least one nucleic acid molecule is integrated into the host cell chromosomal DNA.

By "integrated into the chromosomal DNA" or "chromosomally integrated" is meant herein that the nucleic acid sequence has become incorporated into the chromosomal DNA of the host cell. Typically, chromosomal integration occurs via the process of "homologous recombination," wherein the homologous regions of the introduced (incoming) nucleic acid sequence align with homologous regions of the host chromosome. Subsequently, the sequence between the homologous regions is replaced by the incoming sequence in a double crossover. Thus, "chromosomally integrated" is used interchangeably herein with "homologously recombined" or "homologously integrated."

In a particular embodiment, said at least one nucleic acid molecule is integrated into the host cell chromosomal DNA within a AOX1 locus of a yeast chromosome.

### Kit

The present invention also concerns a kit for producing recombinant mink hair keratin protein comprising:
a) a host cell, and
b) a nucleic acid as defined in the section *"Recombinant nucleic acid molecule encoding a mink hair keratin protein"* above or an expression vector as defined in the section *"Expression vector"* above.

The term "host cell" has herein the same meaning as defined in the section *"Host cell*" above.

In a particular embodiment, said host cell is a bacterial cell or a yeast, as defined in the section *"Host cell"* above.

In a more particular embodiment, said host cell is an *Escherichia coli* bacterial cell, as defined in the section *"Host cell"* above or a *Komagataella phaffii* cell, as defined in the section *"Host cell"* above.

### Production of recombinant mink hair keratin protein

The present invention also concerns a method for producing recombinant mink hair keratin protein, comprising:
a) providing a host cell comprising the nucleic acid as defined in the section *"Recombinant nucleic acid molecule encoding a mink hair keratin protein"* above or the expression vector as defined in the section *"Expression vector"* above, and
b) culturing said host cell under conditions and for a period of time enabling the production of said mink hair keratin protein by said host cell.

The term "host cell" has herein the same meaning as defined in the section *"Host cell"* above.

In a particular embodiment, said host cell is a bacterial cell or a yeast, as defined in the section *"Host cell"* above.

In a more particular embodiment, said host cell is an *Escherichia coli* bacterial cell, as defined in the section *"Host cell"* above or a *Komagataella phaffii* cell, as defined in the section *"Host cell"* above.

In a more particular embodiment, the host cell used in the method for producing recombinant mink hair keratin protein is a host cell of the invention, as defined in the section *"Host cell"* above.

As used herein, the term "culturing" or "cultured" refers to the *in vitro* steps necessary to incubate a population of cells (such as a population of host cells, for example host bacterial cells or host yeast cells) under conditions that support the growth and viability of the cells, as well as the production of proteins of interest by said cells.

The culturing step of the present disclosure can be carried out according to a suitable culture medium and culture conditions known in the art. Further, the culture step includes a batch culture, a continuous culture, and a fed-batch culture.

The medium used for culture shall meet the requirements of the specific host cells used in a proper manner.

For example, sugar sources which may be used for the medium include saccharides and carbohydrates such as glucose, saccharose, lactose, fructose, maltose, starch, cellulose, etc.; oils and fats such as soybean oil, sunflower oil, castor oil, coconut oil, etc.; fatty acids such as palmitic acid, stearic acid, linoleic acid, etc.; glycerol; alcohols such as ethanol; and organic acids such as acetic acid. These substances can be used individually or as a mixture, but are not limited thereto.

For example, carbon sources which may be used for the medium may be crude sucrose or glucose, or molasses containing a large amount of sucrose. Other various carbon sources may be used.

For example, nitrogen sources which may be used for the medium include peptone, yeast extract, beef extract, malt extract, corn steep liquor, soybean meal, and urea or inorganic compounds, for example, ammonium sulfate, ammonium chloride, ammonium phosphate, ammonium carbonate, and ammonium nitrate. The nitrogen sources can also be used individually or as a mixture, but are not limited thereto.

For example, phosphate sources which may be used for the medium include potassium dihydrogen phosphate, dipotassium hydrogen phosphate, or corresponding sodium-containing salts.

Further, the culture medium may contain metallic salts such as magnesium sulfate or iron sulfate. In addition to the above materials, essential growth substances such as amino acids and vitamins may be used. Appropriate precursors may also be used for the culture medium. The raw materials described above may be added batch-wise or continuously to an incubator in an appropriate manner.

A pH of the culture medium may be adjusted by using a basic compound such as sodium hydroxide, potassium hydroxide, and ammonia; or an acidic compound such as phosphoric acid and sulfuric acid in an appropriate manner. Additionally, foam formation may be suppressed by using an antifoaming agent such as fatty acid polyglycol ester. Oxygen or an oxygen-containing gas (e.g., air) may be injected into the culture medium to maintain an aerobic condition.

Specifically, the culturing temperature is preferably between 30° C and 37°C. The culturing may continue until a desired amount of mink hair keratin is obtained.

In a particular embodiment, the production method of the invention further comprises a step c) of collecting the produced mink hair keratin protein.

In a particular embodiment, the collected mink hair keratin protein is in an unfolded state.

As used herein, the terms "protein in unfolded state", "unfolded protein", "protein in misfolded state", "misfolded protein" and "denatured protein" are used interchangeably and refer to a protein which does not possess which does not possess native structures such as secondary, tertiary or quaternary structure where a native structure for the protein does possess one or more such structures, or which possess structures such as secondary, tertiary or quaternary structure, which differ from the native structure of the protein.

The term "folded protein" refers to a protein that possesses stable, native tertiary structure or other structural determinants or three-dimensional structure characteristic of its native form.

In a particular embodiment, said collected mink hair keratin protein is in the form of inclusion bodies.

The term "inclusion bodies" as used herein refers to inactive aggregates of heterologous proteins expressed in prokaryotes or eukaryotes.

As well-known from the skilled person, heterologous proteins, when produced by microorganisms such as bacteria and yeasts, may be produced by the cells in an unfolded or misfolded state and fail to reach their native conformation.

Therefore, in particular embodiment of the production method of the invention, when the collected mink hair keratin protein is in an unfolded state, said method further comprises a step d) of refolding the mink hair keratin protein.

By "refolding" is meant herein that a fully or partially unfolded, misfolded or denatured protein adopts secondary, tertiary and quaternary structure like that of the native protein.

Refolding methods are well-known from the skilled person and typically include dialysis method; dilution method; chromatography methods such as size-exclusion chromatography; methods involving immobilization folding catalyst and artificial chaperones such as zeolite absorbing systems and GroEL-GroES chaperone system; enzyme-assisted refolding methods; and use of chemical additives such as denaturants, protein stabilizers, and protein aggregation inhibitors.

### Recombinant mink hair protein

The present invention also concerns a recombinant mink hair protein obtainable by the method of preparation as defined in the section *"Production of recombinant mink hair keratin protein".*

The present invention further concerns a recombinant protein comprising a recombinant mink hair keratin protein sequence as defined in the section *"Recombinant nucleic acid molecule encoding a mink hair keratin protein"* above, and further comprising:
- a secretion signal, as defined in the section *"Recombinant nucleic acid molecule encoding a mink hair keratin protein"* above, at the N-terminal end of said mink hair keratin protein sequence and/or
- a tag, as defined in the section *"Recombinant nucleic acid molecule encoding a mink hair keratin protein"* above at the N-terminal and/or C-terminal end of said mink hair keratin protein sequence.

In a particular embodiment, said mink hair keratin protein comprises, or consists of, an amino acid sequence at least 80% identical to a sequence selected from the group consisting of SEQ ID NO: 1 [K31], SEQ ID NO: 2 [K35], SEQ ID NO: 3 [K81], SEQ ID NO: 4 [K85], SEQ ID NO: 5 [K32] and SEQ ID NO: 6 [K82]. In a more particular embodiment, said mink hair keratin protein comprises, or consists of, an amino acid sequence at least 85%, at least 90%, at least 95%, at least 96%, at least 97%, at least 98%, or at least 99% identical to a sequence selected from the group consisting of SEQ ID NO: 1 [K31], SEQ ID NO: 2 [K35], SEQ ID NO: 3 [K81], SEQ ID NO: 4 [K85], SEQ ID NO: 5 [K32] and SEQ ID NO: 6 [K82]. In still a particular embodiment, said mink hair keratin protein comprises, or consists of, an amino acid sequence selected from the group consisting of SEQ ID NO: 1 [K31], SEQ ID NO: 2 [K35], SEQ ID NO: 3 [K81], SEQ ID NO: 4 [K85], SEQ ID NO: 5 [K32] and SEQ ID NO: 6 [K82].

In a particular embodiment said mink hair keratin protein sequence comprises at least one mutation removing at least one glycosylation site.

As used herein, the term "glycosylation site" has the same meaning as the one defined in the section *"Recombinant nucleic acid molecule encoding a mink hair keratin protein"* above".

By "mutation" is meant an alteration in the amino acid sequence of the wild-type protein, following one or more modification(s) of the nucleotide sequence encoding said wild-type protein. The mutation may be an addition, a deletion or a substitution of an amino acid by another amino acid relative to the original wild-type amino acid sequence.

In a preferred embodiment, said mutation removing at least one glycosylation site is a substitution. In a more preferred embodiment, said mutation removing at least one glycosylation site is a substitution, preferably by an alanine (A) or by a glutamic acid (E), of the asparagine (N), threonine (T) or serine (S) residue on which glycosylation occurs, or of one of the amino acids signaling the glycosylation site, as defined above.

In a particular embodiment, said glycosylation site is in the keratin protein comprising or consisting of an amino acid sequence at least 85%, at least 90%, at least 95%, at least 96%, at least 97%, at least 98%, at least 99%, or 100% identical to the sequence SEQ ID NO: 6 [K82]. In a more particular embodiment, said mutation removing said glycosylation site is a substitution, preferably by an alanine (A) or by a glutamic acid (E), of the asparagine (N) located at position 437 of the keratin protein comprising or consisting of an amino acid sequence at least 85%, at least 90%, at least 95%, at least 96%, at least 97%, at least 98%, at least 99%, or 100% identical to the sequence SEQ ID NO: 6 [K82], or of an asparagine (N) corresponding to the asparagine located at position 437 of the keratin protein comprising or consisting of an amino acid sequence at least 85%, at least 90%, at least 95%, at least 96%, at least 97%, at least 98%, at least 99%, or 100% identical to the sequence SEQ ID NO: 6 [K82].

In a particularly preferably embodiment, said mink hair keratin protein sequence comprises at least one mutation removing at least one glycosylation site comprises or consists of an amino acid sequence at least 85%, at least 90%, at least 95%, at least 96%, at least 97%, at least 98%, at least 99%, or 100% identical to the sequence SEQ ID NO: 41 [K82 N437A] or to the sequence SEQ ID NO: 42 [K82 N437E], provided that said sequence comprises the mutation N437A or the mutation N437E.

In a particular embodiment, said tag is selected from the group consisting of FLAG tag, HA tag, His tag and c-myc tag. In a more preferred embodiment, the tag is His tag, in particular a single or double insertion of six histidine residues.

In a particular embodiment, the tag, in particular the His tag, is at the N-terminal end of the mink hair keratin protein. In another particular embodiment, the tag, in particular the His tag, is at the C-terminal end of the mink hair keratin protein.

In a particular embodiment, said recombinant protein comprises, or consists of, an amino acid sequence at least 85%, at least 90%, at least 95%, at least 96%, at least 97%, at least 98%, or at least 99% identical to a sequence selected from the group consisting of SEQ ID NO: 7 [His-K31], SEQ ID NO: 8 [His-K35], SEQ ID NO: 9 [His-K81], SEQ ID NO: 10 [His-K85], SEQ ID NO: 11 [His-K32] and SEQ ID NO: 12 [His-K82]. In a more particular embodiment, said recombinant protein comprises, or consists of, an amino acid sequence selected from the group consisting of SEQ ID NO: 7 [His-K31], SEQ ID NO: 8 [His-K35], SEQ ID NO: 9 [His-K81], SEQ ID NO: 10 [His-K85], SEQ ID NO: 11 [His-K32] and SEQ ID NO: 12 [His-K82].

In a particular embodiment, said secretion signal is selected from the group consisting of α factor signal peptide, invertase 2 signal peptide, wild-type UPO signal peptide, killer protein signal peptide, secretion signal of the Kph-0030 protein and a genetic fusion of a pre-alpha sequence to Ost1 co-translocation signal, as defined in the section *"Recombinant nucleic acid molecule encoding a mink hair keratin protein"* above.

In a particular embodiment, said recombinant protein comprises, or consists of, an amino acid sequence at least 85%, at least 90%, at least 95%, at least 96%, at least 97%, at least 98%, or at least 99% identical to a sequence selected from the group consisting of SEQ ID NO: 19 [preostalpha SP-K31], SEQ ID NO: 20 [See prepo alpha SP-K31], SEQ ID NO: 21 [Gma UPO SP-K32], SEQ ID NO: 22 [preostalpha SP-K82], SEQ ID NO: 23 [Killer protein SP-K85] , SEQ ID NO: 24 [Killer protein SP-K35], SEQ ID NO: 25 [alpha factor SP-K81], SEQ ID NO: 26 [0030 SP-K31], SEQ ID NO: 27 [0030 SP-K32], SEQ ID NO: 28 [0030 SP-K82], and SEQ ID NO: 29 [Invertase 2 SP-K85]. In a more particular embodiment, said recombinant protein comprises, or consists of, an amino acid sequence selected from the group consisting of SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, and SEQ ID NO: 29.

In a particularly preferred embodiment, said recombinant protein an amino acid sequence at least 85%, at least 90%, at least 95%, at least 96%, at least 97%, at least 98%, or at least 99% identical to a sequence selected from the group consisting of SEQ ID NO: 30 [preostalpha SP-K31-His2], SEQ ID NO: 31 [See prepo alpha SP-K31-His2], SEQ ID NO: 32 [Gma UPO SP-K32-His2], SEQ ID NO: 33 [preostalpha SP-K82-His2], SEQ ID NO: 34 [Killer protein SP-K85-His2] , SEQ ID NO: 35 [Killer protein SP-K35-His2], SEQ ID NO: 36 [alpha factor SP-K81-His2], SEQ ID NO: 37 [0030 SP-K31-His2], SEQ ID NO: 38 [0030 SP-K32-His2], SEQ ID NO: 39 [0030 SP-K82-His2], SEQ ID NO: 40 [Invertase 2 SP-K85-His2], SEQ ID NO: 43 ("preostalpha SP-KRT82_N437A-His2") and SEQ ID NO: 44 ("preostalpha SP-KRT82_N437E-His2"). In a more particular embodiment, said recombinant protein comprises, or consists of, an amino acid sequence selected from the group consisting of SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43 and SEQ ID NO: 44.

The present invention will be further illustrated by the examples and figures disclosed below.

### BRIEF DESCRIPTION OF THE FIGURES

**Figure 1****:** SDS-PAGE analysis of *E. coli* recombinant *Homo sapiens* (Hs) and *N*. *vison* (Nv) keratins with expected masses: KRT 31 (50.0 kDa), KRT 32 (53.4 kDa) and KRT 35 (53.9 kDa). W: whole cell fraction; S: soluble fraction; IB: purified inclusion bodies.
**Figure 2****:** SDS-PAGE analysis of *E. coli* recombinant *Homo sapiens* (Hs) *N. vison* (Nv) keratins with expected masses: KRT 81 (59.8 kDa), KRT 82 (59.9 kDa) and KRT 85 (59.4 kDa). W: whole cell fraction; S: soluble fraction; IB: purified inclusion bodies.
**Figure 3****:** SDS-PAGE analysis nickel affinity purified *E. coli* produced recombinant *Homo sapiens* (Hs) and *N. vison* (Nv) keratin with expected masses: KRT 31 (50.0 kDa). Ft: column flow through; 50-500: imidazole (mM) stepwise elution gradient; Strip: NaOH cleaning wash.
**Figure 4****:** SDS-PAGE analysis nickel affinity purified *E. coli* produced recombinant *N*. *vison* (Nv) keratins with expected masses: KRT 32 (53.4 kDa) and KRT 82 (59.9 kDa). Ft: column flow through; 50-500: imidazole (mM) stepwise elution gradient; Strip: NaOH cleaning wash.
**Figure 5****:** SDS-PAGE analysis nickel affinity purified *E. coli* produced recombinant *Homo sapiens* (Hs) and *N*. *vison* (Nv) keratins with expected masses: KRT 35 (53.9 kDa) and KRT 81 (59.8 kDa). Ft: column flow through; 50-500: imidazole (mM) stepwise elution gradient; Strip: NaOH cleaning wash.
**Figure 6****:** SDS-PAGE analysis nickel affinity purified *E. coli* produced recombinant *N*. *vison* (Nv) keratins with expected masses: KRT 81 (59.8 kDa) and KRT 85 (59.4 kDa). Ft: column flow through; 50-500: imidazole (mM) stepwise elution gradient; Strip: NaOH cleaning wash.
**Figure 7****:** Western Blot of native (N) and enzymatically deglycosylated (DG) *K. phaffii* secreted recombinant *N. vison* keratin proteins: KRT 31, KRT 32, KRT 82 and KRT 85.
**Figure 8****:** Western Blot of native (N) and deglycosylated (DG) *K. phaffii* secreted recombinant *N. vison* keratin KRT 82 proteins: wild-type (WT) and mutants N96A, N96E, N437A, and N437E.
**Figure 9****:** Western Blot of native (N) and deglycosylated (DG) *K. phaffii* secreted recombinant *N*. *vison* keratin proteins KRT 35 and KRT 81.
**Figure 10****:** Western Blot of individually *K. phaffii* secreted mink keratin protein under methanol induced promoter (pFMD) obtained from the culture media.
**Figure 11****:** Western Blot of individually constitutively expressed *K. phaffii* mink keratin protein shown retained with the cell (cell pellet) and secreted (supernatant).

### BRIEF DESCRIPTION OF THE SEQUENCES

| SEQ ID NO: | Description | Sequence |
|---|---|---|
| 1 | Mink KRT31 protein | MPYNCCLPNVSCRSTCSSRPCVPPSCHTCTLPGACNIPANVGNCGWFCEGSF NGSEKETMQFLNDRLASYLEKVRQLERENAELESRIRERCQQQEPYVCPSYQS YFRTIEELQQKILCAKSENARLVVQIDNAKLAADDFRTKYETELGLRQLVESDI NGLRRILDELTLCKSDLEAQVESLKEELLCLKRNHEEEVNTLRCQIGDRLNVEV DAAPAVDLNRVLNETRCQYEALVETNRRDVEEWFTTQTEELNKQVVSSSEQL QSCQAEIIELRRTVNALEIELQAQHNLRDSLENTLTETEARYSSQLSQLQCMIT NVESQLAEIRSDLERQNQEYQVLLDVRARLECEINTYRGLLESEDCKLPCNPC ATTNACGSSGSCVSNSCAPCTPCVPRPRCGPCNSFVR |
| 2 | Mink KRT35 protein | MASKCLKASFSSGSLRGLGGAGGGSARVSAIYPSSSCKLPSLSRGARSFSACSV GLGRSSSRAAGCLSALCLPAGGFTTSYNLGGGWFGDGILTGSEKETMQSLND RLASYLEKVRQLERENASLEGRIREWCEQQVPYLSPDYQSYFRTIEELQKKTL CTKAENARLVVQIDNAKLAADDFRTKYETELSMRQLVESDMNGLRRILDDLTL CKSDLEAQVESLKEELLCLKKNHEEEVNSLRCQLGDRLNVEVDAAPPVDLNRV LDEMRCQYETLVENNRRDAEEWFNTQTEELNQQVVSSSEQLQSCQAEIIELR RSVNALEIELQAQQSMRDALESTLAETEARYSSQLAQMQCLISNVEAQLAEIRS DLERQNQEYQVLLDVRARLECEINTYRGLLESEEGKLPCNPCAPDHTPGKSC LPCLPAVSCGPGAVRSTCSPRTICVPCPGGRF |
| 3 | Mink KRT81 protein | MTCGSGFCGRAFSCISACGPRPGRCCITAAPYRGVSCYRGLTGGFGSHSVCG GFRAGSCGRSFGYRSGGVCGPSPPCITSVSVNESLLTPLNLEIDPNAQCVKHE EKEQIKCLNSRFAAFIDKVRFLEQQNKLLETKWQFYQNRKCCESNLEPLFEGY IETLRREAECVEADSGRLASELNHVQEVLEGYKKKYEEEVALRATAENEFVAL KKDVDCAYLRKSDLEANAEALTEEIDFLRRLYEEEIRVLHAHISDTSVIVKMDNS RDLNMDCIVAEIKAQYDDIASRSRAEAESWYRSKCEEMKATVIRHGETLRRTK EEINELNRMIQRLTAEVENAKCQNTKLEAAVTQAEQQGETALSDARCKLAELE AALQKAKQDMACLVKEYQEVMNSKLGLDIEIATYRRLLEGEEQRLCEGVGAV NVCVSSSRGGVVCGDLCVSGSRPVTGSACSAPCSGNLAVSTGMCAPCAPCAP CAPCAPCGPCNSVTSCGLGTGGVGSCGISSCGVGSCASSCRRC |
| 4 | Mink KRT85 protein | MSCRSYRISSGCGVTRTFSSCSAVAPKTGNRCCISAAPYRGVSCYRGLTGFGS RSLSNLGSCGPRMAMGGFRAGSCGRSFGYRSGGVCGPSPPCITTVSVNESLL TPLNLEIDPNAQCVKHEEKEQIKCLNNRFAAFIDKVRFLEQQNKLLETKWQFY QNQRCCESNMEPLFNGYIETLRREAECVEADSGRLASELNHVQEVLEGYKKK YEEEVALRATAENEFVVLKKDVDCAYLRKSDLEANVEALVEESSFLKRLYDEEL QVLHAHISDTSVIVKMDNSRDLNMDCVVAEIKAQYDDIASRSRAEAESWYRSK CEEMKATVIRHGETLRRTKEEINELNRMIQRLTAEIENAKCQRAKLEAAVAEAE QQGEAALTDARCKLAELEAALQKAKQDMACLLKEYQEVMNSKLGLDIEIATY RRLLEGEEHRLCEGVGSVNVCVSSSRGGVACGGLTCSTTPGRQIASGPVATG GSITVMAPDSCAPCQPRSSSFSCGSSRSVRFA |
| 5 | Mink KRT32 protein | MTSNCSASIKSCPRPSSVCSSSMSCRPEMCLGYVCQPVSCVPSVSMPTTYRPA SCFSKTYLSSSCWAASGISSSMGTCSWFCEGSFNGNEKETMQSLNDRLACYLE KVRQLERENAELESKIQEACQSQVATMSPDYQSYFRTIEELQQKVLCTKAENA RMVVHIDNAKLAADDFRTKYETELTMRQLVEADTNGLRRILDELTLCKADLEA QVESLKEELLCLKKNHEQEVSALRCQLGDRLNIEVDAAPPVDLNRMLEEMRC QYETVVEANLRDVEEWFNTQMEELNQQVATSSEQLQSYQSDIIDLRRTVNTL EIELQAQHSLRDSLESTLAETEARYGSQLAQMQCLISNVEAQLAEIRCDLERQN QEYKVLLDVKARLECEINTYRGLLESEDCKLPCNPCSTPSCPPCAPSPSMPRT VCVPRTVCVPCVPCPQSRF |
| 6 | Mink KRT82 protein | MSCRSFQLGSRCGGRSFSSCSAVLPRMVTHYAVSKGPCQSGGGSALRALGCL GSRSLCNVGFGRPRVASRCGLPGFGYRVGAPCGSSPCIAPVTINESLLVPLEL EIDPAVQKVKRDEKEQIKCLNNRFASFINKVRFLEQKNKLLETKWNFMQQQR CCQSNIEPIFQAYISALRRQLDCVAGDRARLESELCSLQDVLESYKKKYEEELSL RPCAENEFVALKKDVDTAFLMKADLETNAEALVQEIDFLKGLYEEEICLLQSQI SETSVIVKMDNSRELDVDGIIAEIKAQYDDIASRSKAEAEAWYQSRYEELRLTA GNHCDNLRNRKNEILEMNKLIQRLQQEIENVKAQRCKLEAAVTQAEQQGEAA LSDAKCKLVGLEEALQKAKQDMACLLKEYQEVMNSKLGLDIEIATYRRLLEGE EHRLCEGIGPVNISVSSSKGAVLYEPCVVSTPVLRTGVLKTSGACSVVGTGEV YVPCEPQGLLSCVSTRSSCMKLGAGSSSSHKC |
| 7 | His-KRT31 fusion protein | MGSSHHHHHHSSGLVPRGSHMASMTGGQQMGRDGMPYNCCLPNVSCRST CSSRPCVPPSCHTCTLPGACNIPANVGNCGWFCEGSFNGSEKETMQFLNDR LASYLEKVRQLERENAELESRIRERCQQQEPYVCPSYQSYFRTIEELQQKILCA KSENARLVVQIDNAKLAADDFRTKYETELGLRQLVESDINGLRRILDELTLCKS DLEAQVESLKEELLCLKRNHEEEVNTLRCQIGDRLNVEVDAAPAVDLNRVLNE TRCQYEALVETNRRDVEEWFTTQTEELNKQVVSSSEQLQSCQAEIIELRRTVN ALEIELQAQHNLRDSLENTLTETEARYSSQLSQLQCMITNVESQLAEIRSDLER QNQEYQVLLDVRARLECEINTYRGLLESEDCKLPCNPCATTNACGSSGSCVS NSCAPCTPCVPRPRCGPCNSFVR |
| 8 | His-KRT35 fusion protein | MGSSHHHHHHSSGLVPRGSHMASMTGGQQMGRDGMASKCLKASFSSGSLR GLGGAGGGSARVSAIYPSSSCKLPSLSRGARSFSACSVGLGRSSSRAAGCLSAL CLPAGGFTTSYNLGGGWFGDGILTGSEKETMQSLNDRLASYLEKVRQLEREN ASLEGRIREWCEQQVPYLSPDYQSYFRTIEELQKKTLCTKAENARLVVQIDNA KLAADDFRTKYETELSMRQLVESDMNGLRRILDDLTLCKSDLEAQVESLKEEL LCLKKNHEEEVNSLRCQLGDRLNVEVDAAPPVDLNRVLDEMRCQYETLVENN RRDAEEWFNTQTEELNQQVVSSSEQLQSCQAEIIELRRSVNALEIELQAQQSM RDALESTLAETEARYSSQLAQMQCLISNVEAQLAEIRSDLERQNQEYQVLLDV |
| | | RARLECEINTYRGLLESEEGKLPCNPCAPDHTPGKSCLPCLPAVSCGPGAVRS TCSPRTICVPCPGGRF |
| 9 | His-KRT81 fusion protein | MGSSHHHHHHSSGLVPRGSHMASMTGGQQMGRDGMTCGSGFCGRAFSCIS ACGPRPGRCCITAAPYRGVSCYRGLTGGFGSHSVCGGFRAGSCGRSFGYRS GGVCGPSPPCITSVSVNESLLTPLNLEIDPNAQCVKHEEKEQIKCLNSRFAAFI DKVRFLEQQNKLLETKWQFYQNRKCCESNLEPLFEGYIETLRREAECVEADS GRLASELNHVQEVLEGYKKKYEEEVALRATAENEFVALKKDVDCAYLRKSDLE ANAEALTEEIDFLRRLYEEEIRVLHAHISDTSVIVKMDNSRDLNMDCIVAEIKAQ YDDIASRSRAEAESWYRSKCEEMKATVIRHGETLRRTKEEINELNRMIQRLTAE VENAKCQNTKLEAAVTQAEQQGETALSDARCKLAELEAALQKAKQDMACLV KEYQEVMNSKLGLDIEIATYRRLLEGEEQRLCEGVGAVNVCVSSSRGGVVCG DLCVSGSRPVTGSACSAPCSGNLAVSTGMCAPCAPCAPCAPCAPCGPCNSVT SCGLGTGGVGSCGISSCGVGSCASSCRRC |
| 10 | His-KRT85 fusion protein | MGSSHHHHHHSSGLVPRGSHMASMTGGQQMGRDGMSCRSYRISSGCGVTR TFSSCSAVAPKTGNRCCISAAPYRGVSCYRGLTGFGSRSLSNLGSCGPRMAM GGFRAGSCGRSFGYRSGGVCGPSPPCITTVSVNESLLTPLNLEIDPNAQCVKH EEKEQIKCLNNRFAAFIDKVRFLEQQNKLLETKWQFYQNQRCCESNMEPLFN GYIETLRREAECVEADSGRLASELNHVQEVLEGYKKKYEEEVALRATAENEFV VLKKDVDCAYLRKSDLEANVEALVEESSFLKRLYDEELQVLHAHISDTSVIVKM DNSRDLNMDCVVAEIKAQYDDIASRSRAEAESWYRSKCEEMKATVIRHGETLR RTKEEINELNRMIQRLTAEIENAKCQRAKLEAAVAEAEQQGEAALTDARCKLA ELEAALQKAKQDMACLLKEYQEVMNSKLGLDIEIATYRRLLEGEEHRLCEGVG SVNVCVSSSRGGVACGGLTCSTTPGRQIASGPVATGGSITVMAPDSCAPCQP RSSSFSCGSSRSVRFA |
| 11 | His-KRT32 fusion protein | MGSSHHHHHHSSGLVPRGSHMASMTGGQQMGRDGMTSNCSASIKSCPRPSS VCSSSMSCRPEMCLGYVCQPVSCVPSVSMPTTYRPASCFSKTYLSSSCWAASG ISSSMGTCSWFCEGSFNGNEKETMQSLNDRLACYLEKVRQLERENAELESKIQ EACQSQVATMSPDYQSYFRTIEELQQKVLCTKAENARMVVHIDNAKLAADDF RTKYETELTMRQLVEADTNGLRRILDELTLCKADLEAQVESLKEELLCLKKNH EQEVSALRCQLGDRLNIEVDAAPPVDLNRMLEEMRCQYETVVEANLRDVEEW FNTQMEELNQQVATSSEQLQSYQSDIIDLRRTVNTLEIELQAQHSLRDSLESTL AETEARYGSQLAQMQCLISNVEAQLAEIRCDLERQNQEYKVLLDVKARLECEI NTYRGLLESEDCKLPCNPCSTPSCPPCAPSPSMPRTVCVPRTVCVPCVPCPQS RF |
| 12 | His-KRT82 fusion protein | MGSSHHHHHHSSGLVPRGSHMASMTGGQQMGRDGMSCRSFQLGSRCGGR SFSSCSAVLPRMVTHYAVSKGPCQSGGGSALRALGCLGSRSLCNVGFGRPRV ASRCGLPGFGYRVGAPCGSSPCIAPVTINESLLVPLELEIDPAVQKVKRDEKEQ IKCLNNRFASFINKVRFLEQKNKLLETKWNFMQQQRCCQSNIEPIFQAYISALR RQLDCVAGDRARLESELCSLQDVLESYKKKYEEELSLRPCAENEFVALKKDVD TAFLMKADLETNAEALVQEIDFLKGLYEEEICLLQSQISETSVIVKMDNSRELDV DGIIAEIKAQYDDIASRSKAEAEAWYQSRYEELRLTAGNHCDNLRNRKNEILEM NKLIQRLQQEIENVKAQRCKLEAAVTQAEQQGEAALSDAKCKLVGLEEALQK AKQDMACLLKEYQEVMNSKLGLDIEIATYRRLLEGEEHRLCEGIGPVNISVSSS KGAVLYEPCVVSTPVLRTGVLKTSGACSVVGTGEVYVPCEPQGLLSCVSTRS SCMKLGAGSSSSHKC |
| 13 | α factor signal peptide | MRFPSIFTAVLFAASSALAAPVNTTTEDETAQIPAEAVIGYLDLEGDFDVAVLP FSNSTNNGLLFINTTIASIAAKEEGVSLDKREA |
| 14 | Invertase 2 signal peptide | MLLQAFLFLLAGFAAKISA |
| 15 | Wild-type UPO signal peptide | MRGTPIFASLIALFAHAAIAFPAYGSLAGLTREQLDEILPTLEIRA |
| 16 | Killer protein signal peptide | MTKPTQVLVRSVSILFFITLLHLVVA |
| 17 | secretion signal of the Kph-0030 protein | MKFAISTLLILQAAAVFAA |
| 18 | genetic fusion of a pre-alpha sequence to Ost1 co-translocation signal | MRQVWFSWIVGLFLCFFNVSSAAPVNTTTEDETAQIPAEAVIGYSDLEGDFDV AVLPFSNSTNNGLLFINTTIASIAAKEEGVSLEKREAEA |
| 19 | preostalpha SP-KRT31 fusion protein | MRQVWFSWIVGLFLCFFNVSSAAPVNTTTEDETAQIPAEAVIGYSDLEGDFDV AVLPFSNSTNNGLLFINTTIASIAAKEEGVSLEKREAEAPYNFCLPSLSCRTSCS SRPCVPPSCHSCTLPGACNIPANVSNCNWFCEGSFNGSEKETMQFLNDRLAS YLEKVRQLERDNAELENLIRERSQQQEPLLCPSYQSYFKTIEELQQKILCTKSE NARLVVQIDNAKLAADDFRTKYQTELSLRQLVESDINGLRRILDELTLCKSDLE AQVESLKEELLCLKSNHEQEVNTLRCQLGDRLNVEVDAAPTVDLNRVLNETR SQYEALVETNRREVEQWFTTQTEELNKQVVSSSEQLQSYQAEIIELRRTVNAL EIELQAQHNLRDSLENTLTESEARYSSQLSQVQSLITNVESQLAEIRSDLERQN QEYQVLLDVRARLECEINTYRSLLESEDCNLPSNPCATTNACSKPIGPCLSNPC TSCVPPAPCTPCAPRPRCGPCNSFVR |
| 20 | See prepo alpha SP-KRT31 fusion protein | MRFPSIFTAVLFAASSALAAPVNTTTEDETAQIPAEAVIGYLDLEGDFDVAVLP FSNSTNNGLLFINTTIASIAAKEEGVSLDKREAPYNCCLPNVSCRSTCSSRPCV PPSCHTCTLPGACNIPANVGNCGWFCEGSFNGSEKETMQFLNDRLASYLEKV RQLERENAELESRIRERCQQQEPYVCPSYQSYFRTIEELQQKILCAKSENARLV VQIDNAKLAADDFRTKYETELGLRQLVESDINGLRRILDELTLCKSDLEAQVES LKEELLCLKRNHEEEVNTLRCQIGDRLNVEVDAAPAVDLNRVLNETRCQYEA LVETNRRDVEEWFTTQTEELNKQVVSSSEQLQSCQAEIIELRRTVNALEIELQA QHNLRDSLENTLTETEARYSSQLSQLQCMITNVESQLAEIRSDLERQNQEYQV LLDVRARLECEINTYRGLLESEDCKLPCNPCATTNACGSSGSCVSNSCAPCTP CVPRPRCGPCNSFVR |
| 21 | Gma UPO SP-KRT32 fusion protein | MRGTPIFASLIALFAHAAIAFPAYGSLAGLTREQLDEILPTLEIRATSNCSASIKS CPRPSSVCSSSMSCRPEMCLGYVCQPVSCVPSVSMPTTYRPASCFSKTYLSSS CWAASGISSSMGTCSWFCEGSFNGNEKETMQSLNDRLACYLEKVRQLERENA ELESKIQEACQSQVATMSPDYQSYFRTIEELQQKVLCTKAENARMVVHIDNAK LAADDFRTKYETELTMRQLVEADTNGLRRILDELTLCKADLEAQVESLKEELL CLKKNHEQEVSALRCQLGDRLNIEVDAAPPVDLNRMLEEMRCQYETVVEANL RDVEEWFNTQMEELNQQVATSSEQLQSYQSDIIDLRRTVNTLEIELQAQHSLR DSLESTLAETEARYGSQLAQMQCLISNVEAQLAEIRCDLERQNQEYKVLLDVK ARLECEINTYRGLLESEDCKLPCNPCSTPSCPPCAPSPSMPRTVCVPRTVCVP CVPCPQSRF |
| 22 | preostalpha SP-KRT82 fusion protein | MRQVWFSWIVGLFLCFFNVSSAAPVNTTTEDETAQIPAEAVIGYSDLEGDFDV AVLPFSNSTNNGLLFINTTIASIAAKEEGVSLEKREAEASCRSFQLGSRCGGRS FSSCSAVLPRMVTHYAVSKGPCQSGGGSALRALGCLGSRSLCNVGFGRPRVA SRCGLPGFGYRVGAPCGSSPCIAPVTINESLLVPLELEIDPAVQKVKRDEKEQI KCLNNRFASFINKVRFLEQKNKLLETKWNFMQQQRCCQSNIEPIFQAYISALR RQLDCVAGDRARLESELCSLQDVLESYKKKYEEELSLRPCAENEFVALKKDVD TAFLMKADLETNAEALVQEIDFLKGLYEEEICLLQSQISETSVIVKMDNSRELDV DGIIAEIKAQYDDIASRSKAEAEAWYQSRYEELRLTAGNHCDNLRNRKNEILEM NKLIQRLQQEIENVKAQRCKLEAAVTQAEQQGEAALSDAKCKLVGLEEALQK AKQDMACLLKEYQEVMNSKLGLDIEIATYRRLLEGEEHRLCEGIGPVNISVSSS KGAVLYEPCVVSTPVLRTGVLKTSGACSVVGTGEVYVPCEPQGLLSCVSTRS SCMKLGAGSSSSHKC |
| 23 | Killer protein SP-KRT85 fusion protein | MTKPTQVLVRSVSILFFITLLHLVVASCRSYRISSGCGVTRTFSSCSAVAPKTG NRCCISAAPYRGVSCYRGLTGFGSRSLSNLGSCGPRMAMGGFRAGSCGRSFG YRSGGVCGPSPPCITTVSVNESLLTPLNLEIDPNAQCVKHEEKEQIKCLNNRFA AFIDKVRFLEQQNKLLETKWQFYQNQRCCESNMEPLFNGYIETLRREAECVE ADSGRLASELNHVQEVLEGYKKKYEEEVALRATAENEFVVLKKDVDCAYLRKS DLEANVEALVEESSFLKRLYDEELQVLHAHISDTSVIVKMDNSRDLNMDCVVA EIKAQYDDIASRSRAEAESWYRSKCEEMKATVIRHGETLRRTKEEINELNRMIQ RLTAEIENAKCQRAKLEAAVAEAEQQGEAALTDARCKLAELEAALQKAKQDM ACLLKEYQEVMNSKLGLDIEIATYRRLLEGEEHRLCEGVGSVNVCVSSSRGGV ACGGLTCSTTPGRQIASGPVATGGSITVMAPDSCAPCQPRSSSFSCGSSRSVR FA |
| 24 | Killer protein SP-KRT35 fusion protein | MTKPTQVLVRSVSILFFITLLHLVVAASKCLKASFSSGSLRGLGGAGGGSARVS AIYPSSSCKLPSLSRGARSFSACSVGLGRSSSRAAGCLSALCLPAGGFTTSYNL GGGWFGDGILTGSEKETMQSLNDRLASYLEKVRQLERENASLEGRIREWCEQ QVPYLSPDYQSYFRTIEELQKKTLCTKAENARLVVQIDNAKLAADDFRTKYET ELSMRQLVESDMNGLRRILDDLTLCKSDLEAQVESLKEELLCLKKNHEEEVNSL RCQLGDRLNVEVDAAPPVDLNRVLDEMRCQYETLVENNRRDAEEWFNTQTE ELNQQVVSSSEQLQSCQAEIIELRRSVNALEIELQAQQSMRDALESTLAETEAR YSSQLAQMQCLISNVEAQLAEIRSDLERQNQEYQVLLDVRARLECEINTYRGL LESEEGKLPCNPCAPDHTPGKSCLPCLPAVSCGPGAVRSTCSPRTICVPCPGG RF |
| 25 | alpha factor SP-KRT81 fusion protein | MRFPSIFTAVLFAASSALAAPVNTTTEDETAQIPAEAVIGYLDLEGDFDVAVLP FSNSTNNGLLFINTTIASIAAKEEGVSLDKREATCGSGFCGRAFSCISACGPRP GRCCITAAPYRGVSCYRGLTGGFGSHSVCGGFRAGSCGRSFGYRSGGVCGP |
| | | SPPCITSVSVNESLLTPLNLEIDPNAQCVKHEEKEQIKCLNSRFAAFIDKVRFLE QQNKLLETKWQFYQNRKCCESNLEPLFEGYIETLRREAECVEADSGRLASEL NHVQEVLEGYKKKYEEEVALRATAENEFVALKKDVDCAYLRKSDLEANAEAL TEEIDFLRRLYEEEIRVLHAHISDTSVIVKMDNSRDLNMDCIVAEIKAQYDDIAS RSRAEAESWYRSKCEEMKATVIRHGETLRRTKEEINELNRMIQRLTAEVENAK CQNTKLEAAVTQAEQQGETALSDARCKLAELEAALQKAKQDMACLVKEYQE VMNSKLGLDIEIATYRRLLEGEEQRLCEGVGAVNVCVSSSRGGVVCGDLCVS GSRPVTGSACSAPCSGNLAVSTGMCAPCAPCAPCAPCAPCGPCNSVTSCGL GTGGVGSCGISSCGVGSCASSCRRC |
| 26 | 0030 SP-KRT31 fusion protein | MKFAISTLLILQAAAVFAAPYNCCLPNVSCRSTCSSRPCVPPSCHTCTLPGAC NIPANVGNCGWFCEGSFNGSEKETMQFLNDRLASYLEKVRQLERENAELESR IRERCQQQEPYVCPSYQSYFRTIEELQQKILCAKSENARLVVQIDNAKLAADDF RTKYETELGLRQLVESDINGLRRILDELTLCKSDLEAQVESLKEELLCLKRNHE EEVNTLRCQIGDRLNVEVDAAPAVDLNRVLNETRCQYEALVETNRRDVEEWF TTQTEELNKQVVSSSEQLQSCQAEIIELRRTVNALEIELQAQHNLRDSLENTLT ETEARYSSQLSQLQCMITNVESQLAEIRSDLERQNQEYQVLLDVRARLECEINT YRGLLESEDCKLPCNPCATTNACGSSGSCVSNSCAPCTPCVPRPRCGPCNSF VR |
| 27 | 0030 SP-KRT32 fusion protein | MKFAISTLLILQAAAVFAATSNCSASIKSCPRPSSVCSSSMSCRPEMCLGYVCQ PVSCVPSVSMPTTYRPASCFSKTYLSSSCWAASGISSSMGTCSWFCEGSFNGN EKETMQSLNDRLACYLEKVRQLERENAELESKIQEACQSQVATMSPDYQSYFR TIEELQQKVLCTKAENARMVVHIDNAKLAADDFRTKYETELTMRQLVEADTN GLRRILDELTLCKADLEAQVESLKEELLCLKKNHEQEVSALRCQLGDRLNIEVD AAPPVDLNRMLEEMRCQYETVVEANLRDVEEWFNTQMEELNQQVATSSEQL QSYQSDIIDLRRTVNTLEIELQAQHSLRDSLESTLAETEARYGSQLAQMQCLIS NVEAQLAEIRCDLERQNQEYKVLLDVKARLECEINTYRGLLESEDCKLPCNPC STPSCPPCAPSPSMPRTVCVPRTVCVPCVPCPQSRF |
| 28 | 0030 SP-KRT82 fusion protein | MKFAISTLLILQAAAVFAASCRSFQLGSRCGGRSFSSCSAVLPRMVTHYAVSK GPCQSGGGSALRALGCLGSRSLCNVGFGRPRVASRCGLPGFGYRVGAPCGS SPCIAPVTINESLLVPLELEIDPAVQKVKRDEKEQIKCLNNRFASFINKVRFLEQ KNKLLETKWNFMQQQRCCQSNIEPIFQAYISALRRQLDCVAGDRARLESELCS LQDVLESYKKKYEEELSLRPCAENEFVALKKDVDTAFLMKADLETNAEALVQE IDFLKGLYEEEICLLQSQISETSVIVKMDNSRELDVDGIIAEIKAQYDDIASRSKAE AEAWYQSRYEELRLTAGNHCDNLRNRKNEILEMNKLIQRLQQEIENVKAQRC KLEAAVTQAEQQGEAALSDAKCKLVGLEEALQKAKQDMACLLKEYQEVMNS KLGLDIEIATYRRLLEGEEHRLCEGIGPVNISVSSSKGAVLYEPCVVSTPVLRTG VLKTSGACSVVGTGEVYVPCEPQGLLSCVSTRSSCMKLGAGSSSSHKC |
| 29 | Invertase 2 SP-KRT85 fusion protein | MLLQAFLFLLAGFAAKISASCRSYRISSGCGVTRTFSSCSAVAPKTGNRCCISA APYRGVSCYRGLTGFGSRSLSNLGSCGPRMAMGGFRAGSCGRSFGYRSGGV CGPSPPCITTVSVNESLLTPLNLEIDPNAQCVKHEEKEQIKCLNNRFAAFIDKV RFLEQQNKLLETKWQFYQNQRCCESNMEPLFNGYIETLRREAECVEADSGRL ASELNHVQEVLEGYKKKYEEEVALRATAENEFVVLKKDVDCAYLRKSDLEAN VEALVEESSFLKRLYDEELQVLHAHISDTSVIVKMDNSRDLNMDCVVAEIKAQY DDIASRSRAEAESWYRSKCEEMKATVIRHGETLRRTKEEINELNRMIQRLTAEI ENAKCQRAKLEAAVAEAEQQGEAALTDARCKLAELEAALQKAKQDMACLLK EYQEVMNSKLGLDIEIATYRRLLEGEEHRLCEGVGSVNVCVSSSRGGVACGGL TCSTTPGRQIASGPVATGGSITVMAPDSCAPCQPRSSSFSCGSSRSVRFA |
| 30 | preostalpha SP-KRT31-His2 fusion protein | MRQVWFSWIVGLFLCFFNVSSAAPVNTTTEDETAQIPAEAVIGYSDLEGDFDV AVLPFSNSTNNGLLFINTTIASIAAKEEGVSLEKREAEAPYNFCLPSLSCRTSCS SRPCVPPSCHSCTLPGACNIPANVSNCNWFCEGSFNGSEKETMQFLNDRLAS YLEKVRQLERDNAELENLIRERSQQQEPLLCPSYQSYFKTIEELQQKILCTKSE NARLVVQIDNAKLAADDFRTKYQTELSLRQLVESDINGLRRILDELTLCKSDLE AQVESLKEELLCLKSNHEQEVNTLRCQLGDRLNVEVDAAPTVDLNRVLNETR SQYEALVETNRREVEQWFTTQTEELNKQVVSSSEQLQSYQAEIIELRRTVNAL EIELQAQHNLRDSLENTLTESEARYSSQLSQVQSLITNVESQLAEIRSDLERQN QEYQVLLDVRARLECEINTYRSLLESEDCNLPSNPCATTNACSKPIGPCLSNPC TSCVPPAPCTPCAPRPRCGPCNSFVRSGGSGGGHHHHHHGGSGGSHHHHH HDGGSGGGSTSRDHMVLHEYVNAAGIT |
| 31 | See prepro alpha SP-KRT31-His2 fusion protein | MRFPSIFTAVLFAASSALAAPVNTTTEDETAQIPAEAVIGYLDLEGDFDVAVLP FSNSTNNGLLFINTTIASIAAKEEGVSLDKREAPYNCCLPNVSCRSTCSSRPCV PPSCHTCTLPGACNIPANVGNCGWFCEGSFNGSEKETMQFLNDRLASYLEKV RQLERENAELESRIRERCQQQEPYVCPSYQSYFRTIEELQQKILCAKSENARLV VQIDNAKLAADDFRTKYETELGLRQLVESDINGLRRILDELTLCKSDLEAQVES LKEELLCLKRNHEEEVNTLRCQIGDRLNVEVDAAPAVDLNRVLNETRCQYEA LVETNRRDVEEWFTTQTEELNKQVVSSSEQLQSCQAEIIELRRTVNALEIELQA QHNLRDSLENTLTETEARYSSQLSQLQCMITNVESQLAEIRSDLERQNQEYQV |
| | | LLDVRARLECEINTYRGLLESEDCKLPCNPCATTNACGSSGSCVSNSCAPCTP CVPRPRCGPCNSFVRSGGSGGGHHHHHHGGSGGSHHHHHHDGGSGGGST SRDHMVLHEYVNAAGIT |
| 32 | Gma UPO SP-KRT32-His2 fusion protein | MRGTPIFASLIALFAHAAIAFPAYGSLAGLTREQLDEILPTLEIRATSNCSASIKS CPRPSSVCSSSMSCRPEMCLGYVCQPVSCVPSVSMPTTYRPASCFSKTYLSSS CWAASGISSSMGTCSWFCEGSFNGNEKETMQSLNDRLACYLEKVRQLERENA ELESKIQEACQSQVATMSPDYQSYFRTIEELQQKVLCTKAENARMVVHIDNAK LAADDFRTKYETELTMRQLVEADTNGLRRILDELTLCKADLEAQVESLKEELL CLKKNHEQEVSALRCQLGDRLNIEVDAAPPVDLNRMLEEMRCQYETVVEANL RDVEEWFNTQMEELNQQVATSSEQLQSYQSDIIDLRRTVNTLEIELQAQHSLR DSLESTLAETEARYGSQLAQMQCLISNVEAQLAEIRCDLERQNQEYKVLLDVK ARLECEINTYRGLLESEDCKLPCNPCSTPSCPPCAPSPSMPRTVCVPRTVCVP CVPCPQSRFSGGSGGGHHHHHHGGSGGSHHHHHHDGGSGGGSTSRDHMV LHEYVNAAGIT |
| 33 | preostalpha SP-KRT82-His2 fusion protein | MRQVWFSWIVGLFLCFFNVSSAAPVNTTTEDETAQIPAEAVIGYSDLEGDFDV AVLPFSNSTNNGLLFINTTIASIAAKEEGVSLEKREAEASCRSFQLGSRCGGRS FSSCSAVLPRMVTHYAVSKGPCQSGGGSALRALGCLGSRSLCNVGFGRPRVA SRCGLPGFGYRVGAPCGSSPCIAPVTINESLLVPLELEIDPAVQKVKRDEKEQI KCLNNRFASFINKVRFLEQKNKLLETKWNFMQQQRCCQSNIEPIFQAYISALR RQLDCVAGDRARLESELCSLQDVLESYKKKYEEELSLRPCAENEFVALKKDVD TAFLMKADLETNAEALVQEIDFLKGLYEEEICLLQSQISETSVIVKMDNSRELDV DGIIAEIKAQYDDIASRSKAEAEAWYQSRYEELRLTAGNHCDNLRNRKNEILEM NKLIQRLQQEIENVKAQRCKLEAAVTQAEQQGEAALSDAKCKLVGLEEALQK AKQDMACLLKEYQEVMNSKLGLDIEIATYRRLLEGEEHRLCEGIGPVNISVSSS KGAVLYEPCVVSTPVLRTGVLKTSGACSVVGTGEVYVPCEPQGLLSCVSTRS SCMKLGAGSSSSHKCSGGSGGGHHHHHHGGSGGSHHHHHHDGGSGGGST SRDHMVLHEYVNAAGIT |
| 34 | Killer protein SP-KRT85-His2 fusion protein | MTKPTQVLVRSVSILFFITLLHLVVASCRSYRISSGCGVTRTFSSCSAVAPKTG NRCCISAAPYRGVSCYRGLTGFGSRSLSNLGSCGPRMAMGGFRAGSCGRSFG YRSGGVCGPSPPCITTVSVNESLLTPLNLEIDPNAQCVKHEEKEQIKCLNNRFA AFIDKVRFLEQQNKLLETKWQFYQNQRCCESNMEPLFNGYIETLRREAECVE ADSGRLASELNHVQEVLEGYKKKYEEEVALRATAENEFVVLKKDVDCAYLRKS DLEANVEALVEESSFLKRLYDEELQVLHAHISDTSVIVKMDNSRDLNMDCVVA EIKAQYDDIASRSRAEAESWYRSKCEEMKATVIRHGETLRRTKEEINELNRMIQ RLTAEIENAKCQRAKLEAAVAEAEQQGEAALTDARCKLAELEAALQKAKQDM ACLLKEYQEVMNSKLGLDIEIATYRRLLEGEEHRLCEGVGSVNVCVSSSRGGV ACGGLTCSTTPGRQIASGPVATGGSITVMAPDSCAPCQPRSSSFSCGSSRSVR FASGGSGGGHHHHHHGGSGGSHHHHHHDGGSGGGSTSRDHMVLHEYVNA AGIT |
| 35 | Killer protein SP-KRT35-His2 fusion protein | MTKPTQVLVRSVSILFFITLLHLVVAASKCLKASFSSGSLRGLGGAGGGSARVS AIYPSSSCKLPSLSRGARSFSACSVGLGRSSSRAAGCLSALCLPAGGFTTSYNL GGGWFGDGILTGSEKETMQSLNDRLASYLEKVRQLERENASLEGRIREWCEQ QVPYLSPDYQSYFRTIEELQKKTLCTKAENARLVVQIDNAKLAADDFRTKYET ELSMRQLVESDMNGLRRILDDLTLCKSDLEAQVESLKEELLCLKKNHEEEVNSL RCQLGDRLNVEVDAAPPVDLNRVLDEMRCQYETLVENNRRDAEEWFNTQTE ELNQQVVSSSEQLQSCQAEIIELRRSVNALEIELQAQQSMRDALESTLAETEAR YSSQLAQMQCLISNVEAQLAEIRSDLERQNQEYQVLLDVRARLECEINTYRGL LESEEGKLPCNPCAPDHTPGKSCLPCLPAVSCGPGAVRSTCSPRTICVPCPGG RFSGGSGGGHHHHHHGGSGGSHHHHHHDGGSGGGSTSRDHMVLHEYVNA AGIT |
| 36 | alpha factor SP-KRT81-His2 fusion protein | MRFPSIFTAVLFAASSALAAPVNTTTEDETAQIPAEAVIGYLDLEGDFDVAVLP FSNSTNNGLLFINTTIASIAAKEEGVSLDKREATCGSGFCGRAFSCISACGPRP GRCCITAAPYRGVSCYRGLTGGFGSHSVCGGFRAGSCGRSFGYRSGGVCGP SPPCITSVSVNESLLTPLNLEIDPNAQCVKHEEKEQIKCLNSRFAAFIDKVRFLE QQNKLLETKWQFYQNRKCCESNLEPLFEGYIETLRREAECVEADSGRLASEL NHVQEVLEGYKKKYEEEVALRATAENEFVALKKDVDCAYLRKSDLEANAEAL TEEIDFLRRLYEEEIRVLHAHISDTSVIVKMDNSRDLNMDCIVAEIKAQYDDIAS RSRAEAESWYRSKCEEMKATVIRHGETLRRTKEEINELNRMIQRLTAEVENAK CQNTKLEAAVTQAEQQGETALSDARCKLAELEAALQKAKQDMACLVKEYQE VMNSKLGLDIEIATYRRLLEGEEQRLCEGVGAVNVCVSSSRGGVVCGDLCVS GSRPVTGSACSAPCSGNLAVSTGMCAPCAPCAPCAPCAPCGPCNSVTSCGL GTGGVGSCGISSCGVGSCASSCRRCSGGSGGGHHHHHHGGSGGSHHHHHH DGGSGGGSTSRDHMVLHEYVNAAGIT |
| 37 | 0030 SP-KRT31-His2 fusion protein | MKFAISTLLILQAAAVFAAPYNCCLPNVSCRSTCSSRPCVPPSCHTCTLPGAC NIPANVGNCGWFCEGSFNGSEKETMQFLNDRLASYLEKVRQLERENAELESR IRERCQQQEPYVCPSYQSYFRTIEELQQKILCAKSENARLVVQIDNAKLAADDF |
| | | RTKYETELGLRQLVESDINGLRRILDELTLCKSDLEAQVESLKEELLCLKRNHE EEVNTLRCQIGDRLNVEVDAAPAVDLNRVLNETRCQYEALVETNRRDVEEWF TTQTEELNKQVVSSSEQLQSCQAEIIELRRTVNALEIELQAQHNLRDSLENTLT ETEARYSSQLSQLQCMITNVESQLAEIRSDLERQNQEYQVLLDVRARLECEINT YRGLLESEDCKLPCNPCATTNACGSSGSCVSNSCAPCTPCVPRPRCGPCNSF VRSGGSGGGHHHHHHGGSGGSHHHHHHDGGSGGGSTSRDHMVLHEYVN AAGIT |
| 38 | 0030 SP-KRT32-His2 fusion protein | MKFAISTLLILQAAAVFAATSNCSASIKSCPRPSSVCSSSMSCRPEMCLGYVCQ PVSCVPSVSMPTTYRPASCFSKTYLSSSCWAASGISSSMGTCSWFCEGSFNGN EKETMQSLNDRLACYLEKVRQLERENAELESKIQEACQSQVATMSPDYQSYFR TIEELQQKVLCTKAENARMVVHIDNAKLAADDFRTKYETELTMRQLVEADTN GLRRILDELTLCKADLEAQVESLKEELLCLKKNHEQEVSALRCQLGDRLNIEVD AAPPVDLNRMLEEMRCQYETVVEANLRDVEEWFNTQMEELNQQVATSSEQL QSYQSDIIDLRRTVNTLEIELQAQHSLRDSLESTLAETEARYGSQLAQMQCLIS NVEAQLAEIRCDLERQNQEYKVLLDVKARLECEINTYRGLLESEDCKLPCNPC STPSCPPCAPSPSMPRTVCVPRTVCVPCVPCPQSRFSGGSGGGHHHHHHGG SGGSHHHHHHDGGSGGGSTSRDHMVLHEYVNAAGIT |
| 39 | 0030 SP-KRT82-His2 fusion protein | MKFAISTLLILQAAAVFAASCRSFQLGSRCGGRSFSSCSAVLPRMVTHYAVSK GPCQSGGGSALRALGCLGSRSLCNVGFGRPRVASRCGLPGFGYRVGAPCGS SPCIAPVTINESLLVPLELEIDPAVQKVKRDEKEQIKCLNNRFASFINKVRFLEQ KNKLLETKWNFMQQQRCCQSNIEPIFQAYISALRRQLDCVAGDRARLESELCS LQDVLESYKKKYEEELSLRPCAENEFVALKKDVDTAFLMKADLETNAEALVQE IDFLKGLYEEEICLLQSQISETSVIVKMDNSRELDVDGIIAEIKAQYDDIASRSKAE AEAWYQSRYEELRLTAGNHCDNLRNRKNEILEMNKLIQRLQQEIENVKAQRC KLEAAVTQAEQQGEAALSDAKCKLVGLEEALQKAKQDMACLLKEYQEVMNS KLGLDIEIATYRRLLEGEEHRLCEGIGPVNISVSSSKGAVLYEPCVVSTPVLRTG VLKTSGACSVVGTGEVYVPCEPQGLLSCVSTRSSCMKLGAGSSSSHKCSGGS GGGHHHHHHGGSGGSHHHHHHDGGSGGGSTSRDHMVLHEYVNAAGIT |
| 40 | Invertase 2 SP-KRT85-His2 fusion protein | MLLQAFLFLLAGFAAKISASCRSYRISSGCGVTRTFSSCSAVAPKTGNRCCISA APYRGVSCYRGLTGFGSRSLSNLGSCGPRMAMGGFRAGSCGRSFGYRSGGV CGPSPPCITTVSVNESLLTPLNLEIDPNAQCVKHEEKEQIKCLNNRFAAFIDKV RFLEQQNKLLETKWQFYQNQRCCESNMEPLFNGYIETLRREAECVEADSGRL ASELNHVQEVLEGYKKKYEEEVALRATAENEFVVLKKDVDCAYLRKSDLEAN VEALVEESSFLKRLYDEELQVLHAHISDTSVIVKMDNSRDLNMDCVVAEIKAQY DDIASRSRAEAESWYRSKCEEMKATVIRHGETLRRTKEEINELNRMIQRLTAEI ENAKCQRAKLEAAVAEAEQQGEAALTDARCKLAELEAALQKAKQDMACLLK EYQEVMNSKLGLDIEIATYRRLLEGEEHRLCEGVGSVNVCVSSSRGGVACGGL TCSTTPGRQIASGPVATGGSITVMAPDSCAPCQPRSSSFSCGSSRSVRFASGG SGGGHHHHHHGGSGGSHHHHHHDGGSGGGSTSRDHMVLHEYVNAAGIT |
| 41 | Mink KRT 82 N437A mutant protein | MSCRSFQLGSRCGGRSFSSCSAVLPRMVTHYAVSKGPCQSGGGSALRALGCL GSRSLCNVGFGRPRVASRCGLPGFGYRVGAPCGSSPCIAPVTINESLLVPLEL EIDPAVQKVKRDEKEQIKCLNNRFASFINKVRFLEQKNKLLETKWNFMQQQR CCQSNIEPIFQAYISALRRQLDCVAGDRARLESELCSLQDVLESYKKKYEEELSL RPCAENEFVALKKDVDTAFLMKADLETNAEALVQEIDFLKGLYEEEICLLQSQI SETSVIVKMDNSRELDVDGIIAEIKAQYDDIASRSKAEAEAWYQSRYEELRLTA GNHCDNLRNRKNEILEMNKLIQRLQQEIENVKAQRCKLEAAVTQAEQQGEAA LSDAKCKLVGLEEALQKAKQDMACLLKEYQEVMNSKLGLDIEIATYRRLLEGE EHRLCEGIGPVAISVSSSKGAVLYEPCVVSTPVLRTGVLKTSGACSVVGTGEV YVPCEPQGLLSCVSTRSSCMKLGAGSSSSHKC |
| 42 | Mink KRT 82 N437E mutant protein | MSCRSFQLGSRCGGRSFSSCSAVLPRMVTHYAVSKGPCQSGGGSALRALGCL GSRSLCNVGFGRPRVASRCGLPGFGYRVGAPCGSSPCIAPVTINESLLVPLEL EIDPAVQKVKRDEKEQIKCLNNRFASFINKVRFLEQKNKLLETKWNFMQQQR CCQSNIEPIFQAYISALRRQLDCVAGDRARLESELCSLQDVLESYKKKYEEELSL RPCAENEFVALKKDVDTAFLMKADLETNAEALVQEIDFLKGLYEEEICLLQSQI SETSVIVKMDNSRELDVDGIIAEIKAQYDDIASRSKAEAEAWYQSRYEELRLTA GNHCDNLRNRKNEILEMNKLIQRLQQEIENVKAQRCKLEAAVTQAEQQGEAA LSDAKCKLVGLEEALQKAKQDMACLLKEYQEVMNSKLGLDIEIATYRRLLEGE EHRLCEGIGPVEISVSSSKGAVLYEPCVVSTPVLRTGVLKTSGACSVVGTGEV YVPCEPQGLLSCVSTRSSCMKLGAGSSSSHKC |
| 43 | preostalpha SP-KRT82_N437A-His2 fusion protein | MRQVWFSWIVGLFLCFFNVSSAAPVNTTTEDETAQIPAEAVIGYSDLEGDFDV AVLPFSNSTNNGLLFINTTIASIAAKEEGVSLEKREAEASCRSFQLGSRCGGRS FSSCSAVLPRMVTHYAVSKGPCQSGGGSALRALGCLGSRSLCNVGFGRPRVA SRCGLPGFGYRVGAPCGSSPCIAPVTINESLLVPLELEIDPAVQKVKRDEKEQI KCLNNRFASFINKVRFLEQKNKLLETKWNFMQQQRCCQSNIEPIFQAYISALR RQLDCVAGDRARLESELCSLQDVLESYKKKYEEELSLRPCAENEFVALKKDVD TAFLMKADLETNAEALVQEIDFLKGLYEEEICLLQSQISETSVIVKMDNSRELDV |
| | | DGIIAEIKAQYDDIASRSKAEAEAWYQSRYEELRLTAGNHCDNLRNRKNEILEM NKLIQRLQQEIENVKAQRCKLEAAVTQAEQQGEAALSDAKCKLVGLEEALQK AKQDMACLLKEYQEVMNSKLGLDIEIATYRRLLEGEEHRLCEGIGPVAISVSSS KGAVLYEPCVVSTPVLRTGVLKTSGACSVVGTGEVYVPCEPQGLLSCVSTRS SCMKLGAGSSSSHKCSGGSGGGHHHHHHGGSGGSHHHHHHDGGSGGGST SRDHMVLHEYVNAAGIT |
| 44 | preostalpha SP-KRT82_N437E-His2 fusion protein | MRQVWFSWIVGLFLCFFNVSSAAPVNTTTEDETAQIPAEAVIGYSDLEGDFDV AVLPFSNSTNNGLLFINTTIASIAAKEEGVSLEKREAEASCRSFQLGSRCGGRS FSSCSAVLPRMVTHYAVSKGPCQSGGGSALRALGCLGSRSLCNVGFGRPRVA SRCGLPGFGYRVGAPCGSSPCIAPVTINESLLVPLELEIDPAVQKVKRDEKEQI KCLNNRFASFINKVRFLEQKNKLLETKWNFMQQQRCCQSNIEPIFQAYISALR RQLDCVAGDRARLESELCSLQDVLESYKKKYEEELSLRPCAENEFVALKKDVD TAFLMKADLETNAEALVQEIDFLKGLYEEEICLLQSQISETSVIVKMDNSRELDV DGIIAEIKAQYDDIASRSKAEAEAWYQSRYEELRLTAGNHCDNLRNRKNEILEM NKLIQRLQQEIENVKAQRCKLEAAVTQAEQQGEAALSDAKCKLVGLEEALQK AKQDMACLLKEYQEVMNSKLGLDIEIATYRRLLEGEEHRLCEGIGPVEISVSSS KGAVLYEPCVVSTPVLRTGVLKTSGACSVVGTGEVYVPCEPQGLLSCVSTRS SCMKLGAGSSSSHKCSGGSGGGHHHHHHGGSGGSHHHHHHDGGSGGGST SRDHMVLHEYVNAAGIT |

### EXAMPLES

### Example 1: Identification of key keratin genes with the public Neogale vison genome sequence.

Key keratin genes within the public *Neogale vison* genome sequence were identified by the inventors, using homology-based search comparing to the known gene sequences for known major human hair keratin protein constituents.

Known human cuticular hair keratin, type I and II, proteins were aligned against the American mink (*Neogale vison)* genome for homologous proteins predicted to be the keratin constituents of mink fur.

A set of six candidate mink keratin protein encoding genes in cDNA form were then ordered for custom gene synthesis based on expression patterns in the keratinisation development of human hair.

The protein sequence encoded by these 6 candidates were as followed:
- KRT31 candidate: SEQ ID NO: 1,
- KRT35 candidate: SEQ ID NO: 2,
- KRT81 candidate: SEQ ID NO: 3,
- KRT85 candidate: SEQ ID NO: 4,
- KRT32 candidate: SEQ ID NO: 5, and
- KRT82 candidate: SEQ ID NO: 6.
The DNA sequences for pre-cortex (KRT31, KRT35, KRT81, KRT85) and cuticle keratins (KRT32, KRT82) were then combinatorially assembled, validated, modified and recombinantly expressed for bacterial or yeast production.

### Example 2: Modification and optimization of the DNA sequences of the mink keratin genes for detectable production and affinity-based purification of the resulting proteins expressed in bacteria or yeasts.

The DNA sequences of the mink keratin genes identified in Example 1 were modified and optimized by the inventors for detectable production and affinity-based purification of the resulting proteins when expressed in *E. coli* bacteria and *K. phaffii* yeast hosts.

These modifications included:
(i) genetic construction of the keratin gene open reading frames (ORFs) under the gene expression control of appropriate host elements:
   For plasmid production in *E. coli* bacteria, the DNA sequences of the mink keratin proteins were operably associated with a T7 promotor and a T7 terminator.
   For integrated production in *K. phaffii* yeast, the DNA sequence of the mink keratin proteins were modified to be integrated within the AOX1 gene locus.
(ii) codon-optimization for optimized translation in *E. coli* and *K. phaffii:*
   The mink keratin proteins encoding nucleic acid sequences were codon-optimized for expression in *E. coli* and *K. phaffii.*
(iii) Genetic fusion of a single or repeated hexahistine residue sequences as an amino or carboxyl-terminal tag for simple affinity-based bacterial and yeast production; and
(iv) N-terminal-addition of a set of signal peptide sequences, in yeast (K. phaffii) constructs to direct the secretion of recombinant keratin protein for industrial-scale production:
   The signal peptide sequences of interest, identified using a signal peptide shuffling system were:
   - "Sce_prepro_alpha" (alpha mating factor): α factor signal peptide from *Saccharomyces cerevisiae* of sequence 
MRFPSIFTAVLFAASSALAAPVNTTTEDETAQIPAEAVIGYLDLEGDFDVAVLPFSNSTNNG
LLFINTTIASIAAKEEGVSLDKREA (SEQ ID NO: 13);
   - "Sce-invertase2": invertase 2 signal peptide from *Saccharomyces cerevisiae* of sequence MLLQAFLFLLAGFAAKISA (SEQ ID NO: 14);
   - "Gma-UPO": wild-type UPO signal peptide from *Galerina marginata* of sequence MRGTPIFASLIALFAHAAIAFPAYGSLAGLTREQLDEILPTLEIRA (SEQ ID NO: 15);
   - "Sce-killer protein": Killer protein signal peptide from *Saccharomyces cerevisiae* of sequence MTKPTQVLVRSVSILFFITLLHLVVA (SEQ ID NO: 16);
   - "Kph-0030": secretion signal of the Kph-0030 protein from *Komagataella phaffii* of sequence MKFAISTLLILQAAAVFAA (SEQ ID NO: 17); and
   - "Sce_preOst1_alpha": Genetic fusion of a pre-alpha sequence to Ost1 co-translocation signal from *Saccharomyces cerevisiae* of sequence

MRQVWFSWIVGLFLCFFNVSSAAPVNTTTEDETAQIPAEAVIGYSDLEGDFDVAVLPFSNS
TNNGLLFINTTIASIAAKEEGVSLEKREAEA (SEQ ID NO: 18).

The DNA sequences encoding the following amino acid sequences were thereby obtained:
- Sequences for *K. phaffii* expression:
   ∘ pPAP008 (FMD promoter)- See prepro alpha SP-KRT31_Nv-His2GFP11 in AOX Locus encoding the protein sequence SEQ ID NO: 31;
   ∘ pPAP008 (FMD promoter)- Gma UPO SP-KRT32_Nv-His2GFP11 in AOX Locus encoding the protein sequence SEQ ID NO: 32;
   ∘ pPAP008 (FMD promoter)- preOst alpha SP-KRT82_Nv-His2GFP11 in AOX Locus encoding the protein sequence SEQ ID NO: 33;
   ∘ pPAP008 (FMD promoter)- Killer Protein SP-KRT85_Nv-His2GFP11 in AOX Locus encoding the protein sequence SEQ ID NO: 34;
   ∘ pPAP008 (FMD promoter)- Killer Protein SP-KRT35_Nv-His2GFP11 in AOX Locus encoding the protein sequence SEQ ID NO: 35;
   ∘ pPAP008 (FMD promoter)-preOst alpha SP- KRT82_N437A_Nv-His2GFP11 in AOX Locus encoding the protein sequence SEQ ID NO: 43;
   ∘ pPAP008 (FMD promoter)-preOst alpha SPSP- KRT82_N437E_Nv-His2GFP11 in AOX Locus encoding the protein sequence SEQ ID NO: 44
   ∘ pPAP009 (GAP promoter)-0030 SP- KRT32_Nv-His2GFP11 in AOX Locus encoding the protein sequence SEQ ID NO: 38;
   ∘ pPAP009 (GAP promoter)-0030 SP- KRT82_Nv-His2GFP11 in AOX Locus encoding the protein sequence SEQ ID NO: 39; and
   ∘ pPAP009 (GAP promoter)-Invertase 2 SP- KRT85_Nv-His2GFP11 in AOX Locus encoding the protein sequence SEQ ID NO: 40
- Sequences of *E. coli* expression:
   ∘ pAGAM22082 (T7) HH-T7L-KRT31(KanR) encoding the protein sequence SEQ ID NO: 7;
   ∘ pAGAM22082 (T7) HH-T7L-KRT32 (KanR) encoding the protein sequence SEQ ID NO: 11;
   ∘ pAGAM22082 (T7) HH-T7L-KRT35 (KanR) encoding the protein sequence SEQ ID NO: 8;
   ∘ pAGAM22082 (T7) HH-T7L-KRT81(KanR) encoding the protein sequence SEQ ID NO: 9;
   ∘ pAGAM22082 (T7) HH-T7L-KRT82 (KanR) encoding the protein sequence SEQ ID NO: 12; and
   ∘ pAGAM22082 (T7) HH-T7L-KRT85 (KanR) encoding the protein sequence SEQ ID NO: 10.

The resultant coding sequences of DNA were synthesised and cloned into a shuttle plasmid containing both E. coli and K. phaffii origins of replication for modular Golden-Gate based secretion signal peptide screening in *K. phaffii* (see example 4 below) or a pET28b derived plasmid for inducible T7 expression in BL21 (DE3) *E. coli* cells (ThermoFisher Scientific) (see example 3 below).

### Example 3: Production of mink hair keratin proteins in E. coli

All six mink hair keratin encoding DNA sequences obtained in Example 2 were cloned into a pET28b derivative plasmid under the control of a T7 promotor via BsaI Golden Gate assembly (New England Biolabs, #R3733) with an in-frame addition of an N-terminal Histidine tag and T7 leader sequence.

Plasmids were then transformed into *E. coli* BL21 DE3 competent cells.

Expression and Inclusion body deposition was confirmed using methods adapted from Palmer and Wingfield (2012) *Curr Protoc Protein Sci* 6:6.3.1-6.3.20.

A pre-culture of 10 mL was prepared in LB media with appropriate antibiotics at 37°C, 250 rpm for 16 h.

The pre-culture was then used for inoculation of 50 mL of culture in LB + glucose (0.05%), Glycerol (2%), and lactose (0.2%) media with appropriate antibiotics.

The cell pellet was harvested after 18 hours of culture via centrifugation, then resuspended in 50 mM Tris HCl pH 7.5, 2 mM EDTA, 100 mM NaCl, 1% TritonX-100, 10 µg/mL lysozyme and incubated for 30 min.

The cell suspension was lysed by sonication at 100% amplitude for 4 cycles at 10 s on 10 s off, to shear DNA and reduce viscosity for tight inclusion bodies pellet formation.

The lysed cells were centrifuged at 15,000g for 15 min at 4°C, and the pellet resuspended in 50 mM Tris HCl pH 7.5, 100 mM NaCl, 1% TritonX-100, 2 M Urea, then sonicated for 3 cycles 100% amplitude 10 s on / 10 s off. The resuspended pellet was then centrifuged at 15,000g for 15 min at 4°C. The resultant pellet was washed twice more with the same buffer and stored at -20°C until solubilisation for Ni-NTA IMAC purification.

Inclusion bodies pellet was re-solubilised overnight at 4°C using 15 ml of 20 mM Tris-HCl, pH 7.5, 8 M urea, 10 mM 1,4-Dithioreitol (DTT) on a rotary mixer.

The resolubilized proteins were cleared of aggregates and analysed by SDS-PAGE and Western blot, using:
- as primary antibody (His-Tag): a monoclonal antibody (HIS.H8); Dilution 1:5000 in TBS-Tween milk (5 % milk) - Catalogue: MA1-21315 (Thermo Fisher Scientific),
- as secondary antibody (anti-mouse): a polyclonal, goat anti-mouse IgG (H+L) secondary antibody, HRP; Dilution 1:20,000 in TBS-Tween milk (5 % milk) - Catalog: #31430 (Thermo Fisher Scientific).

HRP signal was detected via chemiluminescence (Thermo Fisher Scientific, Catalogue #34580).

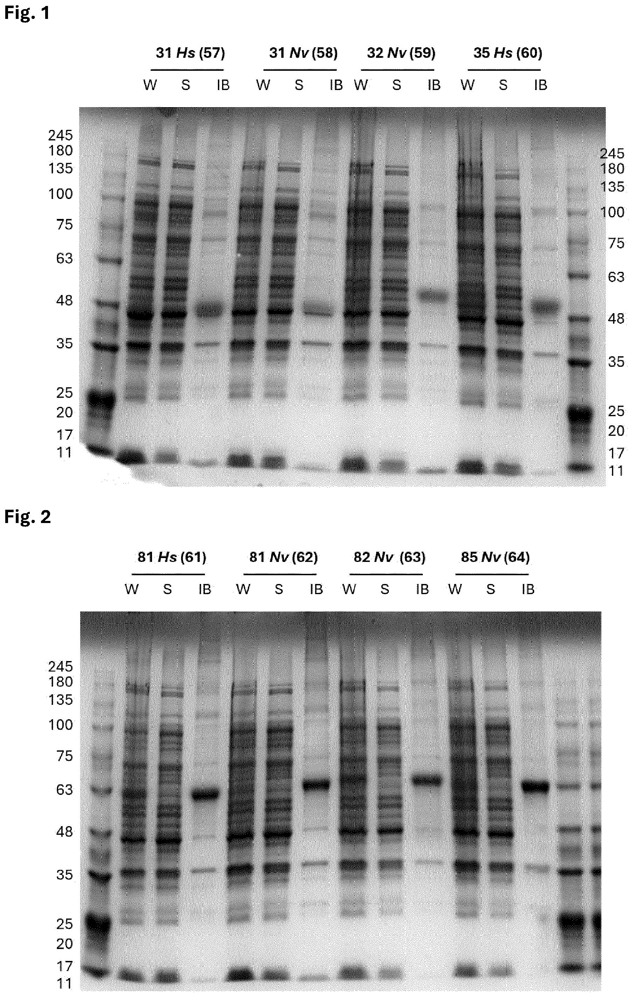

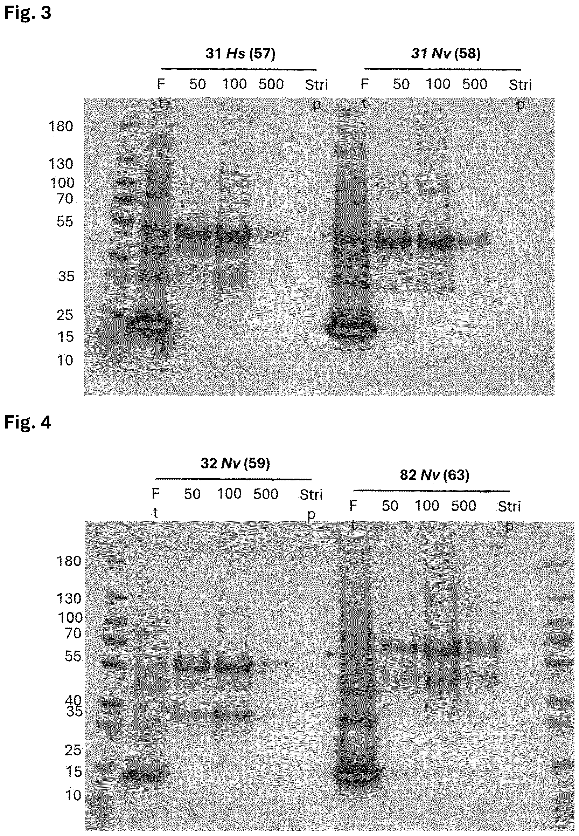

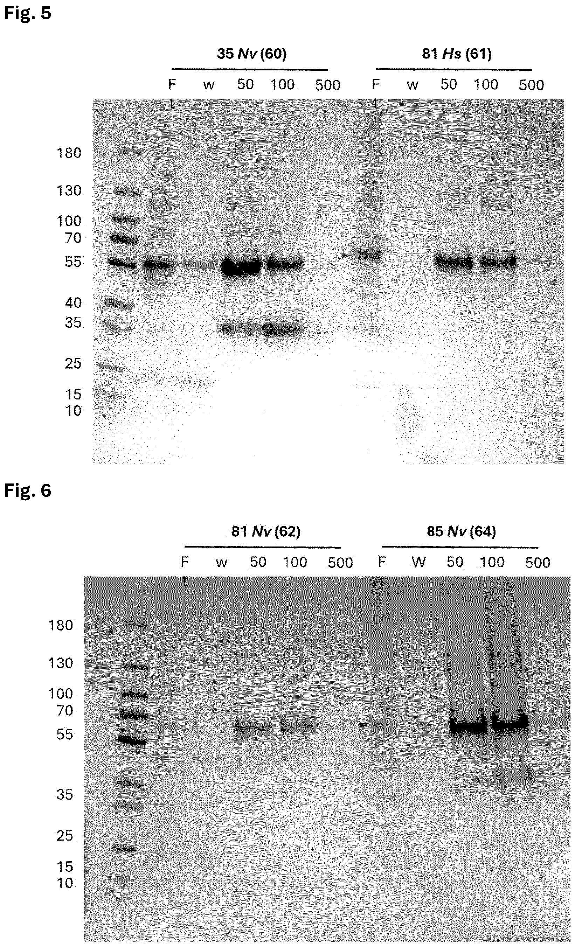

The results of the SDS-PAGE analyses are shown on **Figures 1** to **6****.**

### Example 4: Production of mink hair keratin proteins in K. phaffii

The general approach and respective methods disclosed below were adapted from Püllmann et al. (2021) Communications Biology 4: 562 and Püllmann and Weissenborn (2021) ACS Synth. Biol. 10:1360-1372 using the developed "Yeast secrete and detect Kit" available from Addgene (https://www.addgene.org/kits/marillonnet-yeast-secrete-detect/).

All six mink hair keratin encoding DNA sequences were custom synthesised and cloned as Level 0 gene modules into pAGM9121 plasmid to be screened in a signal peptide shuffling system.

All modules and shuffling with 20 distinct signal peptides were directly cloned in an integrative plasmid backbone (pPAP008 plasmid: FMD promoter (methanol inducible) or pPAP009 plasmid: GAP promoter (constitutive, glucose)). A double His_GFP11 tag was used as C-terminal identification tag for each assembly.

Library preparation was done in *E. coli,* preserving the genetic diversity of the library. Linearisation of the plasmid library DNA was done with AscI/SgsI enzymes.

Subsequent transformation of DNA into *K. phaffii* X-33 strain (ThermoFisher Scientific, catalogue # C18000) was done by electroporation with integration of the DNA into the 3' AOX1 locus via homologous recombination.

Respective *K. phaffii* colonies were screened in 96 well plate setups in 500 µL of liquid media (buffered rich media) respectively.
pPAP008 (FMD promotor): 0.5 % glycerol and 1.5 % methanol as primary carbon source
pPAP009 (GAP promotor): 2 % glucose as primary carbon source
96 well plate cultures were incubated for 72 h cultivation (700 rpm, 30°C)

100 µL of clarified supernatant was transferred for Western/antibody dot blotting to PVDF membrane to detect if the desired protein was produced. The Western/antibody dot blotting was performed using:
- as primary antibody (His-Tag): a monoclonal antibody (HIS.H8); Dilution 1:5000 in TBS-Tween milk (5 % milk) - Catalogue: MA1-21315 (Thermo Fisher Scientific);
- as secondary antibody (anti-mouse): a polyclonal, goat anti-mouse IgG (H+L) secondary antibody, HRP; Dilution 1:20,000 in TBS-Tween milk (5 % milk) - Catalogue: #31430 (Thermo Fisher Scientific).

HRP signal was detected via chemiluminescence (Thermo Fisher Scientific, Catalogue #34580).

After identification of suitable production constructs from the tested library of yeast strains, genomic DNA of the promising strains was extracted (using the protocol described in Lõoke et al. (2011) Biotechniques 50(5):325-8) and then PCR was used to amplify the open reading frames of the heterologous DNA. DNA sequencing was then used to identify the DNA sequence composition of the strains.

Potential glycosylation of candidate keratin proteins in *K. phaffi* was identified by SDS-PAGE analysis (BioRad, #4561094) showing higher mass than expected. Keratin sequences were analysed for N-linked glycosylation sites via PNGase F treatment. Verified glycosylated keratin candidate coding sequences were mutated to neutral alanine bases at possible N-linked glycosylation sequences. The outlined transformation and clonal screen protocols described above were then repeated looking for good production of protein, now with the correct mass.

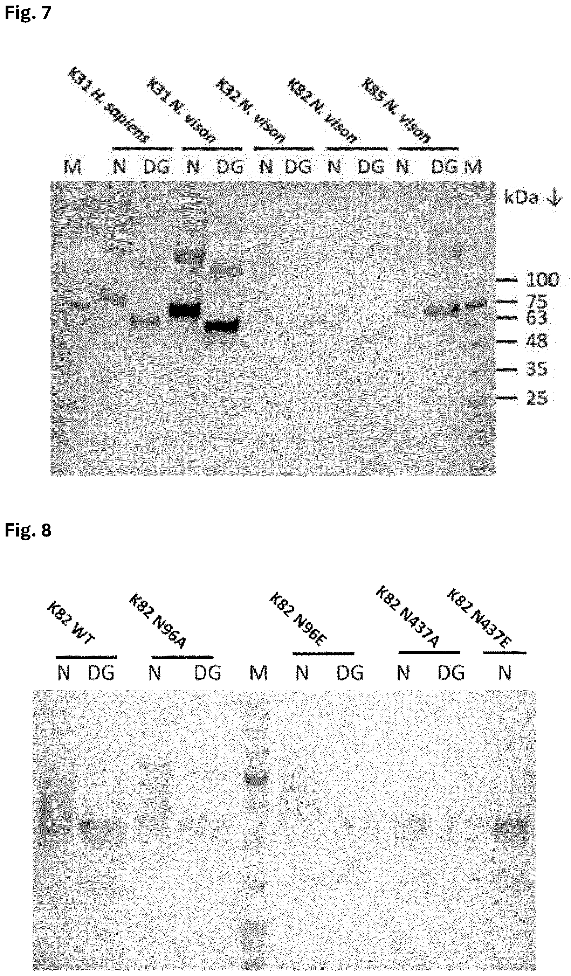

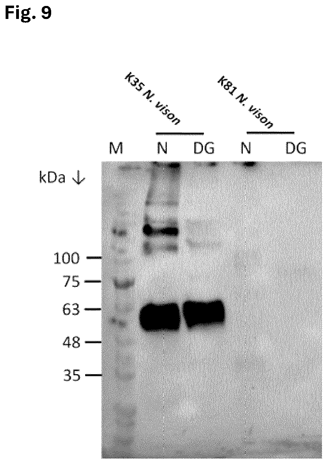

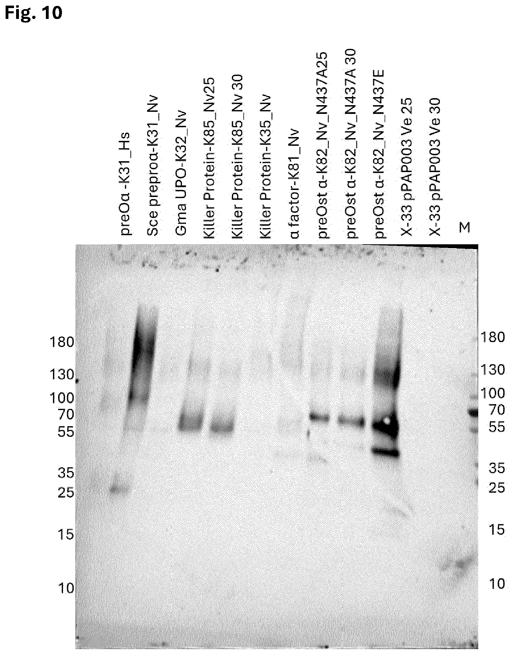

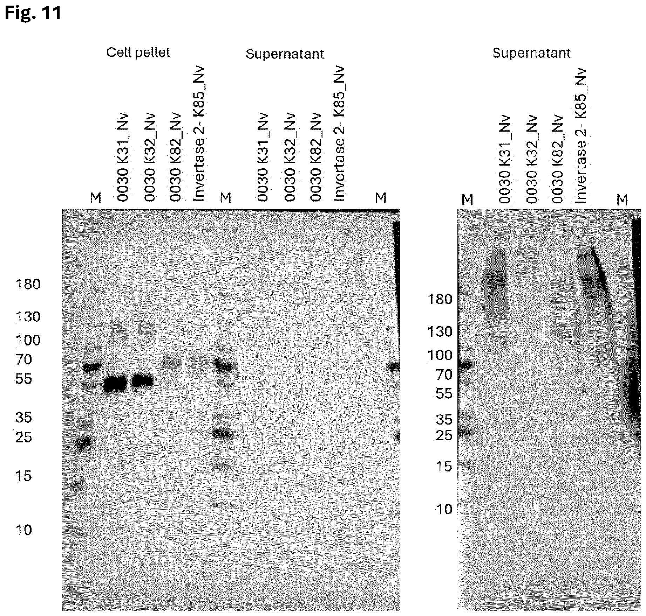

The results of the Western Blot analyses are shown on **Figures 7** to **11****.**

### References

1. Palmer and Wingfield (2012) Preparation and extraction of insoluble (inclusion-body) proteins from Escherichia coli. Curr Protoc Protein Sci 6:6.3.1-6.3.20.
2. Püllmann et al. (2021) A modular two yeast species secretion system for the production and preparative application of unspecific peroxygenases. Communications Biology 4: 562.
3. Püllmann and Weissenborn (2021) Improving the Heterologous Production of Fungal Peroxygenases through an Episomal Pichia pastoris Promoter and Signal Peptide Shuffling System. ACS Synth. Biol. 10:1360-1372.
4. Lõoke et al. (2011) Extraction of genomic DNA from yeasts for PCR-based applications. Biotechniques 50(5):325-8.

## Claims

1. An isolated recombinant nucleic acid molecule comprising at least one nucleic acid sequence encoding at least one mink hair keratin protein and further comprising a heterologous promoter controlling the expression of said at least one nucleic acid sequence.

2. The nucleic acid molecule according to claim 1, wherein said nucleic acid sequence is codon-optimized for prokaryotic expression and/or yeast expression.

3. The nucleic acid molecule according to claim 1 or 2, wherein said nucleic acid sequence is optimized to remove glycosylation sites.

4. The nucleic acid molecule according to any one of claims 1 to 3, wherein said mink hair keratin protein comprises an amino acid sequence at least 80% identical to a sequence selected from the group consisting of SEQ ID NO: 1 (KRT31), SEQ ID NO: 2 (KRT35), SEQ ID NO: 3 (KRT81) and SEQ ID NO: 4 (KRT85).

5. The nucleic acid molecule according to any one of claims 1 to 3, wherein said mink hair keratin protein comprises an amino acid sequence at least 80% identical to a sequence selected from the group consisting of SEQ ID NO: 5 (KRT32) and SEQ ID NO: 6 (KRT82).

6. The nucleic acid molecule according to any one of claims 1 to 5, further comprising a nucleic acid sequence encoding a tag at the N-terminal and/or C-terminal end of the mink cuticular keratin protein.

7. The nucleic acid molecule according to any one of claims 1 to 6, further comprising a nucleic acid sequence encoding a secretion signal at the N-terminal end of the mink cuticular keratin protein.

8. An expression vector comprising at least a nucleic acid molecule according to any one of claims 1 to 7.

9. A host cell comprising at least one nucleic acid molecule according to any one of claims 1 to 7 or an expression vector according to claim 8.

10. A kit for producing recombinant mink hair keratin protein comprising:
a) a host cell, and
b) a nucleic acid molecule according to any one of claims 1 to 7 or an expression vector according to claim 8.

11. A method for producing recombinant mink hair keratin protein comprising:
a) providing a host cell comprising the nucleic acid molecule according to any one of claims 1 to 7 or the expression vector according to claim 8,
b) culturing said host cell under conditions and for a period of time enabling the production of said mink hair keratin protein by said host cell.

12. A recombinant mink hair keratin protein obtainable by the method according to claim 11.

13. A recombinant protein comprising a recombinant mink hair keratin protein sequence, and further comprising:
- a secretion signal at the N-terminal end of said mink hair keratin protein sequence and/or
- a tag at N-terminal and/or C-terminal end of said mink hair keratin protein sequence.

14. The recombinant protein according to claim 13, wherein said mink hair keratin protein sequence comprises at least one mutation removing at least one glycosylation site.

15. The recombinant protein according to claim 13 or 14, wherein said recombinant protein comprises a sequence selected from the group consisting of SEQ ID NO: 30 [preostalpha SP-K31-His2], SEQ ID NO: 31 [See prepo alpha SP-K31-His2], SEQ ID NO: 32 [Gma UPO SP-K32-His2], SEQ ID NO: 33 [preostalpha SP-K82-His2], SEQ ID NO: 34 [Killer protein SP-K85-His2] , SEQ ID NO: 35 [Killer protein SP-K35-His2], SEQ ID NO: 36 [alpha factor SP-K81-His2], SEQ ID NO: 37 [0030 SP-K31-His2], SEQ ID NO: 38 [0030 SP-K32-His2], SEQ ID NO: 39 [0030 SP-K82-His2], SEQ ID NO: 40 [Invertase 2 SP-K85-His2], SEQ ID NO: 43 ("preostalpha SP-KRT82_N437A-His2") and SEQ ID NO: 44 ("preostalpha SP-KRT82_N437E-His2").
